# EUROPEAN PATENT APPLICATION

(11) **EP 1 916 240 A1**
(43) Date of publication of application: **30.04.2008**
(21) Application number: 06022285.8
(22) Date of filing: 25.10.2006
(51) Int. Cl.: C07D 237/08, C07D 237/12, C07D 237/14, C07D 237/18, C07D 237/04, C07D 405/04, C07D 409/04, C07D 401/04, C07D 403/04, C07D 413/04, C07D 417/04, C07D 401/12, C07D 409/12, C07D 307/60

(54) **Pyridazine derivatives**

(71) Applicant: Syngenta Participations AG, 4058 Basel (CH)
(72) Inventor: The designation of the inventor has not yet been filed
(74) Representative: Arunasalam, Velautha-Cumaran

(57) **Abstract**

The present invention relates to novel pyridazine derivatives of formula I as active ingredients which have microbiocidal activity, in particular fungicidal activity : 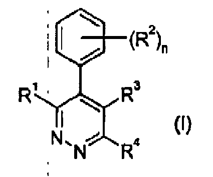 wherein
R¹ is hydrogen, C₁-C₆alkyl, C₁-C₆haloalkyl or C₃-C₆cydoalkyl;
R² is cycloalkyl, cycloalkylalkyl, halocycloalkyl, cycloalkoxy, halocycloalkoxy, cycloalkylalkoxy, halocycloalkylalkoxy, alkoxyalkyl, cycloalkoxyalkoxyalkyl, haloalkoxyalkyl, trialkylsilyl, alkylthioalkyl, haloalkylthioalkyl, cycloalkylthio, halocycloalkylthio, cycloalkylalkylthio, halocycloalkylalkylthio, alkylsulfinylalkyl, alkylsulfonylalkyl, alkylsulfinyl, haloalkylsulfinyl, alkylsulfonyl, haloalkylsulfonyl, optionally substituted arylsulfonyl, optionally substituted aryloxy, optionally substituted heteroaryloxy, optionally substituted arylthio, optionally substituted hoteroarylthio, or
R² together with an adjacent carbon atom forms a fused aromatic carbocyclic ring, a fused non aromatic carbocyclic ring, a fused aromatic heterocyclic ring or a fused non aromatic heterocyclic ring;
R³ is an optionally substituted aryl;
R⁴ is hydrogen, halogen, C₁-C₆alkyl, C₁-C₆haloalkyl, C₁-C₆alkoxy, C₁-C₆haloalkoxy, hydroxy or cyano; and
n is a whole number from 1 to 4.

## Description

The present invention relates to novel pyridazine derivatives as active ingredients which have microbiocidal activity, in particular fungicidal activity. The invention also relates to preparation of these active ingredients, to novel heterocyclic derivatives used as intermediates in the preparation of these active ingredients, to preparation of these novel intermediates, to agrochemical compositions which comprise at least one of the novel active ingredients, to preparation of these compositions and to use of the active ingredients or compositions in agriculture or horticulture for controlling or preventing infestation of plants, harvested food crops, seeds or non-living materials by phytopathogenic microorganisms, preferably fungi.

The present invention provides a compound of formula I: 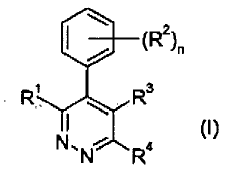 wherein
R¹ is hydrogen, C₁-C₆alkyl, C₁-C₆haloalkyl or C₃-C₆cycloalkyl;
R² is cycloalkyl, cycloalkylalkyl, halocycloalkyl, cycloalkoxy, halocycloalkoxy, cycloalkylalkoxy, halocycloalkylalkoxy, alkoxyalkyl, cycloalkoxyalkoxyalkyl, haloalkoxyalkyl, trialkylsilyl, alkylthioalkyl, haloalkylthioalkyl, cycloalkylthio, halocycloalkylthio, cycloalkylalkylthio, halocycloalkylalkylthio, alkylsulfinylalkyl, alkylsulfonylalkyl, alkylsulfinyl, haloalkylsulfinyl, alkylsulfonyl, haloalkylsulfonyl, optionally substituted arylsulfonyl, optionally substituted aryloxy, optionally substituted heteroaryloxy, optionally substituted arylthio, optionally substituted heteroarylthio; or
R² together with an adjacent carbon atom forms a fused aromatic carbocyclic ring, a fused non aromatic carbocyclic ring, a fused aromatic heterocyclic ring or a fused non aromatic heterocyclic ring;
R³ is an optionally substituted aryl;
R⁴ is hydrogen, halogen, C₁-C₆alkyl, C₁-C₆haloalkyl, C₁-C₆alkoxy, C₁-C₆haloalkoxy, hydroxy or cyano; and
n is a whole number from 1 to 4.

When n is 2, 3 or 4, then each R² independently of each other and independently at each occurrence can be the same or different.

In the above definition aryl includes aromatic hydrocarbon rings like phenyl, naphthyl, anthracenyl, phenanthrenyl and biphenyl, with phenyl being preferred.

Heteroaryl stands for aromatic ring systems comprising mono-, bi- or tricyclic systems wherein at least one oxygen, nitrogen or sulfur atom is present as a ring member. Examples are furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, indazolyl, benzotriazolyl, benzothiazolyl, benzoxazolyl, quinolinyl, isoquinolinyl, phthalazinyl, quinoxalinyl, quinazolinyl, cinnolinyl and naphthyridinyl.

The above carbocyclic ring, heterocyclic ring, aryl group and heteroaryl group may be optionally substituted. This means that they may carry one or more identical or different substituents. Normally not more than three substituents are present at the same time. Examples of substituents of aryl or heteroaryl groups are: halogen, alkyl, haloalkyl, cycloalkyl, cycloalkylalkyl, alkenyl, haloalkenyl, cycloalkenyl, alkynyl, haloalkynyl, alkyloxy, haloalkyloxy, cycloalkoxy, alkenyloxy, haloalkenyloxy, alkynyloxy, haloalkenyloxy, alkylthio, haloalkylthio, cycloalkylthio, alkenylthio, alkynylthio, alkylcarbonyl, haloalkylcarbonyl, cycloalkylcarbonyl, alkenylcarbonyl, alkynylcarbonyl, alkoxyalkyl, cyano, nitro, hydroxy, mercapto, amino, alkylamino, dialkylamino. Typical examples for optionally substituted aryl include 2-fluorophenyl, 2-chlorophenyl, 2-trifluoromethylphenyl, 2-methylphenyl, 2,3-difluorophenyl, 2,4-difluorophenyl, 2,5-difluorophenyl, 2,6-difluorophenyl, 2,3-dichlorophenyl, 2,4-dichlorophenyl, 2,5-dichlorophenyl, 2,6-dichlorophenyl, 2-chloro-3-fluorophenyl, 2-chloro-4-fluorophenyl, 2-chloro-5-fluorophenyl, 2-chloro-6-fluorophenyl, 3-chloro-2-fluorophenyl, 4-chloro-2-fluorophenyl, 5-chloro-2-fluorophenyl, 2-fluoro-3-trifluoromethylphenyl, 2-fluoro-4-trifluoromethylphenyl, 2-fluoro-5-trifluoromethylphenyl, 2-fluoro-6-trifluoromethylphenyl, 2-chloro-3-trifluoromethylphenyl, 2-chloro-4-trifluoromethylphenyl, 2-chloro-5-trifluoromethylphenyl, 2-Chloro-6-trifluoromethylphenyl, 4-fluoro-2-trifluoromethylphenyl, 4-chloro-2-trifluoromethylphenyl, 2-fluoro-3-methylphenyl, 2-fluoro-4-methylphenyl, 2-fluoro-5-methylphenyl, 2-fluoro-6-methylphenyl, 2-chloro-3-methylphenyl, 2-chloro-4-methylphenyl, 2-chloro-5-methylphenyl, 2-chloro-6-methylphenyl, 4-fluoro-2-methylphenyl, 4-chloro-2-methylphenyl, 2,4,6-trifluorophenyl, 2,3,6-trifluorophenyl, 2,3,4-trifluorophenyl, 2,4,6-trichlorophenyl, 2,3,6-trichlorophenyl, 2,3,4-trichlorophenyl, 2,6-difluoro-4-methoxyphenyl, 2,6-difluoro-4-trifluoromethoxyphenyl, 2,6-difluoro-4-trifluoromethylphenyl, 2,6-difluoro-4-cyanophenyl, 2,6-difluoro-4-methylphenyl, 2,6-dichloro-4-methoxyphenyl, 2,6-dichloro-4-trifluoromethoxyphenyl, 2,6-dichloro-4-trifluoromethylphenyl, 2,6-dichloro-4-cyanophenyl, 2,6-dichloro-4-methylphenyl, pentafluorophenyl. Typical examples for optionally substituted heteroaryl include 2-chloro-thiophen-5-yl, 2-bromothiophen-5-yl, 2-methylthiophen-5-yl, 5-chlorothiophen-2-yl, 4-bromo-5-methylthiophen-2-yl, 4-bromothiophen-2-yl, 5-bromothiophen-2-yl, 5-methylthiophen-2-yl, 5-bromofuran-2-yl, 4,5-dimethylfuran-2-yl, 5-methylfuran-2-yl, 5-chlorofuran-2-yl, 3-methylisothiazol-4-yl, 5-methylisoxazol-3-yl, 2-chloropyridin-5-yl, 2-methylpyridin-5-yl, 2-bromopyridin-5-yl, 5-chloropyridin-2-yl, 5-methylpyridin-2-yl, 5-bromopyridin-2-yl, 6-chloropyridin-2-yl, 6-methylpyridin-2-yl, 6-chloropyridin-3-yl, 6-bromopyridin-3-yl, 5-bromopyridin-3-yl, 6-methylpyridin-3-yl, 6-methoxypyridin-3-yl, 5,6-dichloropyridin-3-yl, 2-chloropyridin-4-yl, 2-methylpyridin-4-yl, 2,6-dichloropyridin-4-yl, 2-methylpyrimidin-4-yl.

In the above definition halogen is fluorine, chlorine, bromine or iodine.

The alkyl, alkenyl or alkynyl radicals may be straight-chained or branched.

Alkyl on its own or as part of another substituent is, depending upon the number of carbon atoms mentioned, for example, methyl, ethyl, propyl, butyl, pentyl, hexyl and the isomers thereof, for example, isopropyl, isobutyl, sec-butyl, tert-butyl, isopentyl or tert-pentyl.

A haloalkyl group may contain one or more identical or different halogen atoms and, for example, may stand for CH₂Cl, CHCl₂, CCl₃, CH₂F, CHF₂, CF₃, CF₃CH₂, CH₃CF₂, CF₃CF₂ or CCl₃CCl₂.

Cycloalkyl on its own or as part of another substituent is, depending upon the number of carbon atoms mentioned, for example, cyclopropyl, cyclobutyl, cyclopentyl or cyclohexyl.

Alkenyl on its own or as part of another substituent is, depending upon the number of carbon atoms mentioned, for example, ethenyl, allyl, 1-propenyl, buten-2-yl, buten-3-yl, penten-1-yl, penten-3-yl, hexen-1-yl or 4-methyl-3-pentenyl.

Alkynyl on its own or as part of another substituent is, depending upon the number of carbon atoms mentioned, for example, ethynyl, propyn-1-yl, propyn-2-yl, butyn-1-yl, butyn-2-yl, 1-methyl-2-butynyl, hexyn-1-yl or 1-ethyl-2-butynyl.

The presence of one or more possible asymmetric carbon atoms in a compound of formula I means that the compounds may occur in optically isomeric, that means enantiomeric or diastereomeric forms. As a result of the presence of a possible aliphatic C=C double bond, geometric isomerism, that means cis-trans or (E)-(Z) isomerism may also occur. Also atropisomers may occur as a result of restricted rotation about a single bond. Formula I is intended to include all those possible isomeric forms and mixtures thereof. The present invention intends to include all those possible isomeric forms and mixtures thereof for a compound of formula I.

In each case, the compounds of formula I according to the invention are in free form or in an agronomically usable salt form.

In a fist embodiment, compounds of formula I according to the invention have R¹ which is C₁-C₆alkyl or C₁-C₆haloalkyl.

In a second embodiment, compounds of formula I according to the invention have R² which is cycloalkyl, cycloalkylalkyl, halocycloalkyl, cycloalkoxy, cycloalkylalkoxy, alkoxyalkyl, haloalkoxyalkyl, trialkylsilyl, alkylthioalkyl, haloalkylthioalkyl, cycloalkylthio, cycloalkylalkylthio, alkylsulfinyl, haloalkylsulfinyl, alkylsulfonyl, haloalkylsulfonyl, optionally substituted arylsulfonyl, optionally substituted aryloxy, optionally substituted heteroaryloxy, optionally substituted arylthio or optionally substituted heteroarylthio.

In a third embodiment, compounds of formula I according to the invention have R² which together with an adjacent carbon atom forms a fused aromatic carbocyclic ring, a fused non aromatic carbocyclic ring, a fused aromatic heterocyclic ring or a fused non aromatic heterocyclic ring.

In a fourth embodiment, compounds of formula I according to the invention have R³ which is an optionally substituted aryl.

In a fifth embodiment, compounds of formula I according to the invention have R⁴ which is halogen, C₁-C₈alkyl, C₁-C₈haloalkyl, C₁-C₈alkoxy, C₁-C₆haloalkoxy or hydroxyl.

Preferred subgroups of compounds of formula I according to the invention are those wherein R¹ is C₁-C₆alkyl or C₁-C₆haloalkyl;
R² is cycloalkyl, cycloalkylalkyl, halocycloalkyl, cycloalkoxy, cycloalkylalkoxy, alkoxyalkyl, haloalkoxyalkyl, trialkylsilyl, alkylthioalkyl, haloalkylthioalkyl, cycloalkylthio, cycloalkylalkylthio, alkylsulfinyl, haloalkylsulfinyl, alkylsulfonyl, haloalkylsulfonyl, optionally substituted arylsulfonyl, optionally substituted aryloxy, optionally substituted heteroaryloxy, optionally substituted arylthio or optionally substituted heteroarylthio; or
R² together with an adjacent carbon atom forms a fused aromatic carbocyclic ring, a fused non aromatic carbocyclic ring, a fused aromatic heterocyclic ring or a fused non aromatic heterocyclic ring;
R³ is an optionally substituted aryl;
R⁴ is halogen, C₁-C₆alkyl, C₁-C₆haloalkyl, C₁-C₆alkoxy, C₁-C₆haloalkoxy or hydroxy; and
n is a whole number from 1 to 4.

More preferred subgroups of compounds of formula I according to the invention are those wherein
R¹ is C₁-C₃alkyl;
R² is cycloalkyl, halocycloalkyl, cycloalkoxy, alkoxyalkyl, haloalkoxyalkyl, alkylsulfonyl, optionally substituted arylsulfonyl, optionally substituted aryloxy; or
R², together with the adjacent carbon atom forms a fused aromatic carbocyclic ring, a fused non aromatic carbocyclic ring or a fused aromatic heterocyclic ring;
R³ is an optionally substituted aryl;
R⁴ is halogen, C₁-C₃alkyl, C₁-C₃alkoxy or hydroxy; and
n is a whole number from 1 to 3.

Most preferred subgroups of compounds of formula I according to the invention are those wherein
R¹ is methyl;
R² is cycloalkyl, halocycloalkyl, alkoxyalkyl, haloalkoxyalkyl, alkylsulfonyl, optionally substituted aryloxy; or
R², together with the adjacent carbon atom, forms a fused aromatic carbocyclic ring, a fused non aromatic carbocyclic ring;
R³ is an optionally substituted phenyl;
R⁴ is fluoro, chloro, methyl, methoxy or hydroxy; and
n is a whole number from 1 to 2.

Preferred individual compounds are:
3-chloro-5-(4-cyclopropylphenyl)-6-methyl-4-(2,4,6-trifluorophenyl)-pyridazine,
3-chloro-5-(4-methanesulfonylphenyl)-6-methyl-4-(2,4,6-trifluorophenyl)-pyridazine,
3-chloro-6-methyl-5-naphthalen-2-yl-4-(2,4,6-trifluorophenyl)-pyridazine,
3-chloro-5-[4-(4-chlorophenoxy)-phenyl]-6-methyl-4-(2,4,6-trifluorophenyl)-pyridazine,
3-chloro-6-methyl-5-(4-phenoxyphenyl)-4-(2,4,6-trifluorophenyl)-pyridazine,
3-chloro-6-methyl-5-(4-phenylsulfanylphenyl)-4-(2,4,6-trifluorophenyl)-pyridazine,
3-chloro-5-[4-(4-chlorophenylsulfanyl)-phenyl]-6-methyl-4-(2,4,6-trifluorophenyl)-pyridazine,
3-chloro-6-methyl-5-(4-trifluoromethanesulfonylphenyl)-4-(2,4,6-trifluorophenyl)-pyridazine,
3-chloro-5-(4-cyclopentyloxyphenyl)-6-methyl-4-(2,4,6-trifluorophenyl)-pyridazine,
3-chloro-5-(4-methoxymethylphenyl)-6-methyl-4-(2,4,6-trifluorophenyl)-pyridazine,
3-chloro,-6-methyl-5-(4-methylsutfanylmethylphenyl)-4-(2,4,r)-trifluorophenyl)-pyridazine, and
3-chloro-5-(4-cyclopropylmethylphenyl)-6-methyl-4-(2,4,6-trifluorophenyl)-pyridazine.

Certain pyridazine derivatives with a halogen-, alkyl-, alkoxy, alkylthio-, nitro- and cyano-substituted phenyl group in position 5 have been proposed for controlling plant-destructive fungi, for example in WO 2005/121104 and WO 2006/001175. However, the action of those preparations is not satisfactory in all aspects of agricultural needs. Surprisingly, with the compounds of formula I, new kinds of fungicides having a high level of biological actitivity have now been found.

The compounds of formula 1.2, wherein R¹, R², R³ and n are as defined for compound of formula I and R⁵ is C₁-C₆alkyl or C₁-C₆haloalkyl, can be obtained by reaction of a compound of formula I.1, wherein R¹, R², R³ and n are as defined for compound of formula I and Hal is halogen, preferably fluorine, chlorine or bromine, with an alcohol R⁵OH, wherein R⁵ is C₁-C₆alkyl or C₁-C₆haloalkyl, and a base or with a sodium alkoxide NaOR⁵, wherein R⁵ is C₁-C₆alkyl or C₁-C₆haloalkyl.

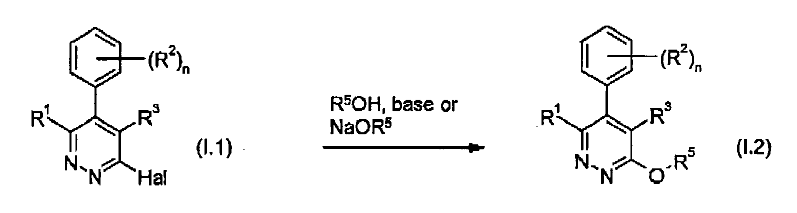

The compounds of formula 1.3, wherein R¹, R², R³ and n are as defined for compound of formula I and R⁶ is C₁-C₆alkyl, can be obtained by transformation of a compound of formula I-1, wherein R¹, R², R³ and n are as defined for compound of formula I and Hal is halogen, preferably chlorine or bromine, with a Grignard reagent R⁶MgHal, wherein R⁶ is C₁-C₈alkyl and Hal is halogen, preferably chlorine or bromine, in the presence of a transition metal catalyst.

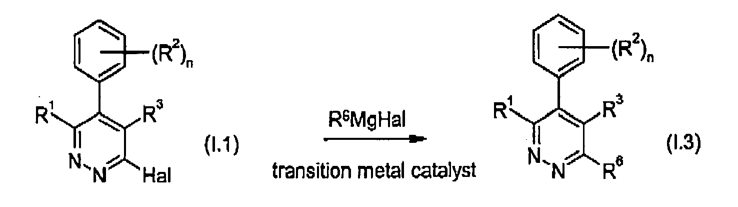

The compounds of formula 1.4, wherein R¹, R², R³ and n are as defined for compound of formula I, can be obtained by transformation of a compound of formula I.1, wherein R¹, R², R³ and n are as defined for compound of formula I and Hal is halogen, preferably chlorine or bromine, with an inorganic fluoride, e.g. potassium fluoride.

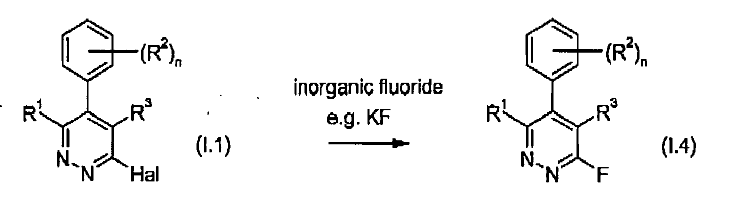

The compounds of formula I.1, wherein R¹, R², R³ and n are as defined for compound of formula I and Hal is halogen, preferably chlorine or bromine, can be obtained by reaction of a compound of formula I.5, wherein R¹, R², R³ and n are as defined for compound of formula I, with a phosphorus oxyhalide, e.g. phosphorus oxychloride or phosphorus oxybromide, or thionyl halide, e.g. thionyl chloride or thionyl bromide.

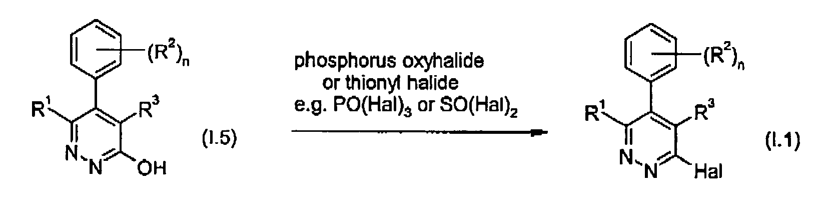

The compounds of formula 1.5, wherein R¹, R², R³ and n are as defined for compound of formula I, can be obtained by reaction of a compound of formula II, wherein R¹, R², R³ and n are as defined for compound of formula I, with a hydrazine derivative, e.g. hydrazine hydrate.

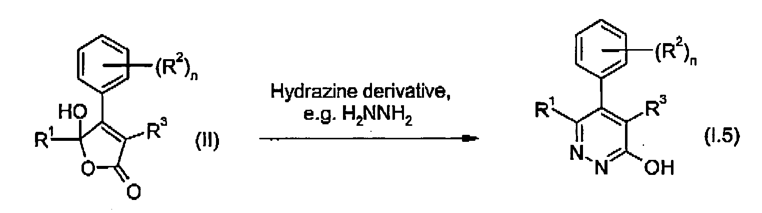

The compounds of formula II, wherein R¹, R², R³ and n are as defined for compound of formula I, can be obtained by oxidation of a compound af formula III, wherein R¹, R², R³ and n are as defined for compound of formula I, with oxygen, air or 3-chloroperbenzoic acid.

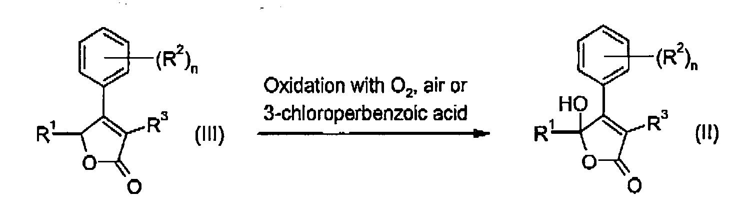

The compounds of formula III, wherein R¹, R², R³ and n are as defined for compound of formula I, can be obtained by reaction of a compound of formula IV, wherein R¹, R², R³ and n are as defined for compound of formula I, with a base, e.g. pyridine, triethylamine, diisopropylethylamine, 1,5-diazabicyclo[4.3.0]non-5-ene or 1,8-diazabicyclo[5.4.0]undec-7-ene.

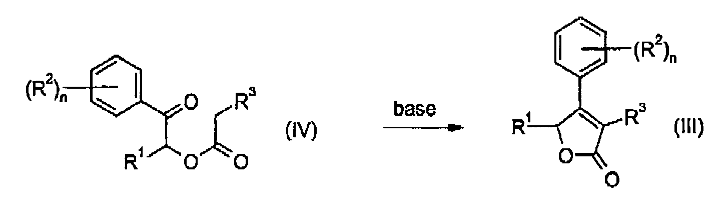

The compounds of formula IV, wherein R¹, R², R³ and n are as defined for compound of formula l, can be obtained by reaction of a compound of formula V, wherein R¹, R² and n are as defined for compound of formula I and Hal is halogen, preferably chlorine or bromine, with a compound of formula VI, wherein R³ is as defined for compound of formula I, and a base, e.g. pyridine, triethylamine, diisopropylethylamine, 1,5-diazabicyclo[4.3.0]non-5-ene or 1,8-diazabicyclo[5.4.0]undec-7-ene.

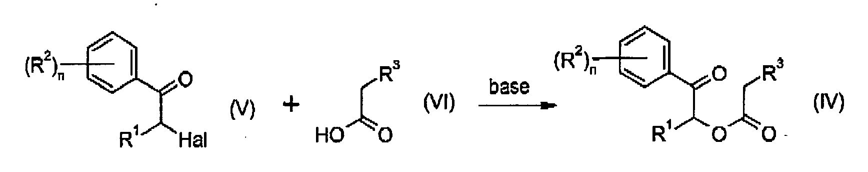

Surprisingly, it has now been found that the novel compounds of formula I have, for practical purposes, a very advantageous spectrum of activities for protecting plants against diseases that are caused by fungi as well as by bacteria and viruses.

The compounds of formula I can be used in the agricultural sector and related fields of use as active ingredients for controlling plant pests or on non-living materials for control of spoilage microorganisms or organisms potentially harmfull to man. The novel compounds are distinguished by excellent activity at low rates of application, by being well tolerated by plants and by being environmentally safe. They have very useful curative, preventive and systemic properties and are used for protecting numerous cultivated plants. The compounds of formula I can be used to inhibit or destroy the pests that occur on plants or parts of plants (fruit, blossoms, leaves, stems, tubers, roots) of different crops of useful plants, while at the same time protecting also those parts of the plants that grow later e.g. from phytopathogenic microorganisms.

It is also possible to use compounds of formula I as dressing agents for the treatment of plant propagation material, *e.g.*, seed, such as fruits, tubers or grains, or plant cuttings (for example rice), for the protection against fungal infections as well as against phytopathogenic fungi occurring in the soil. The propagation material can be treated with a composition comprising a compound of formula I before planting: seed, for example, can be dressed before being sown. The active ingredients according to the invention can also be applied to grains (coating), either by impregnating the seeds in a liquid formulation or by coating them with a solid formulation. The composition can also be applied to the planting site when the propagation material is being planted, for example, to the seed furrow during sowing. The invention relates also to such methods of treating plant propagation material and to the plant propagation material so treated.

Furthermore the compounds according to present invention can be used for controlling fungi in related areas, for example in the protection of technical materials, including wood and wood related technical products, in food storage, in hygiene management.

In addition, the invention could be used to protect non-living materials from fungal attack, e.g. lumber, wall boards and paint.

The compounds of formula I are, for example, effective against the phytopathogenic fungi of the following classes: Fungi imperfecti (e.g. *Botrytis* spp., *Alternaria* spp.) and Basidiomycetes (e.g. *Rhizoctonia* spp., *Hemileia* spp., *Puccinia* spp., *Phakopsora* spp., *Ustilago spp., Tilletia spp*.). Additionally, they are also effective against Ascomycetes (e.g. *Venturia* spp., *Blumeria* spp., *Podosphaera leucotricha, Monilinia* spp., *Fusarium* spp., *Uncinula* spp., *Mycosphaerella* spp., *Pyrenophora* spp., *Rhynchosporium secalis, Magnaporthe* spp., *Colletotrichum* spp., *Gaeumannomyces graminis, Tapesia spp., Ramularia spp., Microdochium nivale, Sclerotinia spp*.) and Oomycetes (e.g. *Phytophthora* spp., *Pythium* spp., *Plasmopara* spp., *Pseudoperonospora cubensis*). Outstanding activity has been observed against powdery mildews (e.g. *Uncinula necator*), rusts (e.g. *Puccinia* spp.) and leaf spots (e.g. *Septoria tritici*). Furthermore, the novel compounds of formula I are effective against phytopathogenic bacteria and viruses (e.g. against *Xanthomonas* spp, *Pseudomonas* spp, *Erwinia amylovora* as well as against the tobacco mosaic virus).

Within the scope of present invention, target crops to be protected typically comprise the following species of plants: cereal (wheat, barley, rye, oat, rice, maize, sorghum and related species); beet (sugar beet and fodder beet); pomes, drupes and soft fruit (apples, pears, plums, peaches, almonds, cherries, strawberries, raspberries and blackberries); leguminous plants (beans, lentils, peas, soybeans); oil plants (rape, mustard, poppy, olives, sunflowers, coconut, castor oil plants, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamomum, camphor) or plants such as tobacco, nuts, coffee, eggplants, sugar cane, tea, pepper, vines, hops, bananas and natural rubber plants, as well as turf and ornamentals.

The target crops in accordance with the invention include conventional as well as genetically enhanced or engineered varieties such as, for example, insect resistant (e.g. Bt. and VIP varieties) as well as disease resistant, herbicide tolerant (e.g. glyphosate- and glufosinate-resistant maize varieties commercially available under the trade names RoundupReady® and LibertyLink®) and nematode tolerant varieties. By way of example, suitable genetically enhanced or engineered crop varieties include the Stoneville 5599BR cotton and Stoneville 4892BR cotton varieties.

The compounds of formula I are used in unmodified form or, preferably, together with the adjuvants conventionally employed in the art of formulation. To this end they are conveniently formulated in known manner to emulsifiable concentrates, coatable pastes, directly sprayable or dilutable solutions or suspensions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compounds of formula I are normally used in the form of compositions and can be applied to the crop area or plant to be treated, simultaneously or in succession with further compounds. These further compounds can be e.g. fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The compounds of formula I are normally used in the form of fungicidal compositions for controlling or protecting against phytopathogenic microorganisms, comprising as active ingredient at least one compound of formula I, in free form or in agrochemically usable salt form, and at least one of the above-mentioned adjuvants.

The compounds of formula I can be mixed with other fungicides, resulting in some cases in unexpected synergistic activities. Mixing components which are particularly preferred are:
Azoles, such as azaconazole, BAY 14120, bitertanol, bromuconazole, cyproconazole, difenoconazole, diniconazole, epoxiconazole, fenbuconazole, fluquinconazole, flusilazole, flutriafol, hexaconazole, imazalil, imibenconazole, ipconazole, metconazole, myclobutanil, pefurazoate, penconazole, prothioconazole, pyrifenox, prochloraz, propiconazole, simeconazole, tebuconazole, tetraconazole, triadimefon, triadimenol, triflumizole, triticonazole;
Pyrimidinyl carbinoles, such as ancymidol, fenarimol, nuarimol;
2-amino-pyrimidines, such as bupirimate, dimethirimol, ethirimol;
Morpholines, such as dodemorph, fenpropidine, fenpropimorph, spiroxamine, tridemorph;
Anilinopyrimidines, such as cyprodinil, mepanipyrim, pyrimethanil;
Pyrroles, such as fenpiclonil, fludioxonil;
Phenylamides, such as benalaxyl, furalaxyl, metalaxyl, R-metalaxyl, ofurace, oxadixyl;
Benzimidazoles, such as benomyl, carbendazim, debacarb, fuberidazole, thiabendazole;
Dicarboximides, such as chlozolinate, dichlozoline, iprodione, myclozoline, procymidone, vinclozoline;
Carboxamides, such as boscalid, carboxin, fenfuram, flutolanil, mepronil, oxycarboxin, penthiopyrad, thifluzamide; guanidines, such as guazatine, dodine, iminoctadine;
Strobilurines, such as azoxystrobin, dimoxystrobin, enestroburin, fluoxastrobin, kresoxim-methyl, metominostrobin, trifloxystrobin, orysastrobin, picoxystrobin, pyraclostrobin;
Dithiocarbamates, such as ferbam, mancozeb, maneb, metiram, propineb, thiram, zineb, ziram;
N-halomethylthlotetrahydrophthalimides, such as captafol, captan, dichlofluanid, fluoromides, folpet, tolyfluanid;
Cu-compounds, such as Bordeaux mixture, copper hydroxide, copper oxychloride, copper sulfate, cuprous oxide, mancopper, oxine-copper;
Nitrophenol-derivatives, such as dinocap, nitrothal-isopropyl;
Organo-phosphorus-derivatives, such as edifenphos, iprobenphos, isoprothiolane, phosdiphen, pyrazophos, tolclofos-methyl;
Pyridazine-derivatives which are known and may be prepared by methods as described in WO 05/121104 and WO 06/001175, such as 3-chloro-5-(4-chloro-phenyl)-6-methyl-4-(2,4,6-trifluoro-phenyl)-pyridazine (formula P.1) and 3-chloro-6-methyl-5-p-tolyl-4-(2,4,6-trifluoro-phenyl)-pyridazine (formula P.2); 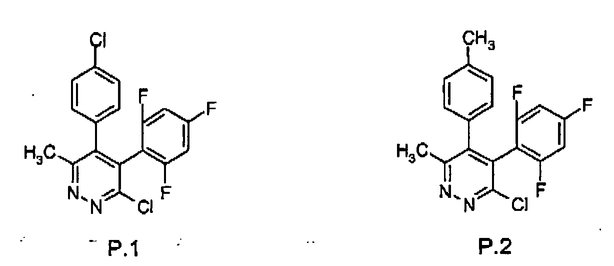
Triazolopyrimidine-derivatives which are known and may be prepared by methods as described in WO98/46607, such as 5-chloro-7-(4-methyl-piperidin-1-yl)-6-(2,4,6-trifluorophenyl)- [1,2,4]triazolo[1,5-a]pyrimidine (formula T.1); 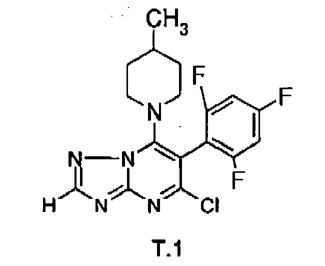
Carboxamide-derivatives which are known and may be prepared by methods as described in WO04/035589 and in WO06/37632, such as 3-difluoromethyl-1-methyl-1H-pyrezole-4-carboxylic acid (9-isopropyp-1,2,3,4-tetrahaydro-1,4-methano-naphthalen-5-yl)-amide (formula U.1); 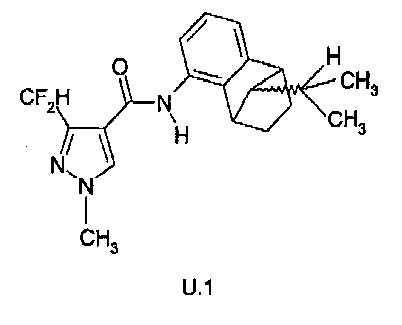
Benzamide-derivatives which are known and may be prepared by methods as described in O 2004/016088, such as N-{-2-[3-chloro-5-(trifluoromethyl)-2-pyridinyl]ethyl}-2-trifluoromethylbenzamide (formula V.1); 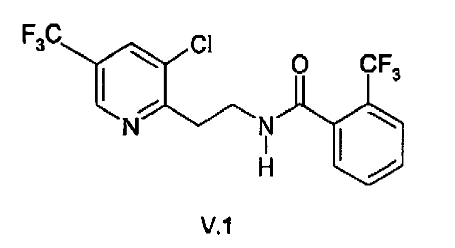 and
Various others, such as acibenzolar-S-methyl, anilazine, benthiavalicarb, blasticidin-S, chinomethionate, chloroneb, chlorothalonil, cyflufenamid, cymoxanil, dichlone, diclocymet, diclomezine, dicloran, diethofencarb, dimethomorph, flumorph, dithianon, ethaboxam, etridiazole, famoxadone, fenamidone, fenoxanil, fentin, ferimzone, fluazinam, fluopicolide, flusulfamide, fenhexamid, fosetyl-aluminium, hymexazol, iprovalicarb, cyazofamid, kasugamycin, mandipropamid, methasulfocarb, metrafenone, nicobifen, pencycuron, phthalide, polyoxins, probenazole, propamocarb, proquinazid, pyroquilon, quinoxyfen, quintozene, sulfur, tiadinil, triazoxide, tricyclazole, triforine, validamycin, zoxamide, glyphosate in form of its monobasic, dibasic or tribasic salts, such as monosodium, monopotassium, monoammonium, mono(dimethylammonium), mono(ethanolammonium), mono(isopropylammonium) and mono(trimethylsulfonium) salts as well as mixtures thereof.

A method of controlling or preventing an infestation of crop plants, harvested food crops or of non-living materials by phytopathogenic or spoilage microorganisms or organisms potentially harmful to man, especially fungal organisms, which comprises the application of a compound of formula I as active ingredient to the plants, to parts of the plants or to the locus thereof, to seeds or or to any part of the non-living materials. Controlling or preventing means reducing the infestation of crop plants or of non-living materials by phytopathogenic or spoilage microorganisms or organisms potentially harmful to man, especially fungal organisms, to such a level that an improvement is demonstrated.

A preferred method of controlling or preventing an infestation of crop plants by phytopathogenic microorganisms, especially fungal organisms, which comprises the application of a compound of formula I, or an agrochemical composition which contains at least one of said compounds, is foliar application. The frequency of application and the rate of application will depend on the risk of infestation by the corresponding pathogen. However, the compounds of formula I can also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula I may also be applied to seeds (coating) by impregnating the seeds or tubers either with a liquid formulation of the fungicide or coating them with a solid formulation.

A formulation [that is, a composition containing the compound of formula I] and, if desired, a solid or liquid adjuvant or monomers for encapsulating the compound of formula I, is prepared in a known manner, typically by intimately mixing and/or grinding the compound with extenders, for example solvents, solid carriers and, optionally, surface active compounds (surfactants).

The agrochemical formulations will usually contain from 0.1 to 99% by weight, preferably from 0.1 to 95% by weight, of the compound of formula I, 99.9 to 1% by weight, preferably 99.8 to 5% by weight, of a solid or liquid adjuvant, and from 0 to 25% by weight, preferably from 0.1 to 25% by weight, of a surfactant.

Advantageous rates of application are normally from 5g to 2kg of active ingredient (a.i.) per hectare (ha), preferably from 10g to 1kg a.i./ha, most preferably from 20g to 600g a.i./ha. When used as seed drenching agent, convenient dosages are from 10mg to 1g of active substance per kg of seeds.

Whereas it is preferred to formulate commercial products as concentrates, the end user will normally use dilute formulations.

The following non-limiting examples illustrate the above-described invention in more detail.

### Example 1: This example illustrates the preparation of 3-chloro-6-methyl-5-naphthalen-2-yl-4-(2,4,6-trifluorophenyl)-pyridazine (Compound No.I.ae.198)

a) Preparation of 2-bromo-1-naphthalen-2-yl-propan-1-one
   Bromine (2.2 ml) is added to the mixture of 1-naphthalen-2-yl-propan-1-one (7.6 g), 0.1 ml of hydrobromic acid (48 % solution) and 100 ml of acetic acid at 0 °C under a nitrogen atmosphere. Subsequently, the mixture is stirred for 2.0 h at room temperature. The reaction mixture is evaporated under reduced pressure. After chromatography on silica gel, using a mixture of heptane / ethyl acetate 9 : 1 as eluent, 2-bromo-1-naphthalen-2-yl-propan-1-one is obtained as a yellow oil.
b) Preparation of 5-hydroxy-5-methyl-4-naphthalen-2-yl-3-(2,4,6-trifluorophenyl)-5H-furan-2-one (Compound No. II.ae.040)
   A mixture of 2-bromo-1-naphthalen-2-yl-propan-1-one (3.1 g), 2,4,6-trifluorophenylacetic acid (2.5 g), 1.3 ml of triethylamine and 30 ml of acetonitrile is stirred for 16 h at room temperature. Subsequently 30 ml of acetonitrile and 4.3 ml of 1,8-diazabicyclo[5.4.0]undec-7-ene (DBU) are added under cooling and stirring is continued for further 3 h. Then air is blown through the reaction mixture for 3 h. An aqueous ammonium chloride solution is added and the mixture is extracted with ethyl acetate. The combined organic layer is washed with a saturated aqueous sodium bicarbonate solution and with brine, dried over sodium sulfate and evaporated under reduced pressure. The remainder is purified by chromatography on silica gel, using a mixture of heptane / ethyl acetate 9 : 1 as eluent to obtain 5-hydroxy-5-methyl-4-naphthalen-2-yl-3-(2,4,6-trifluorophenyl)-5H-furan-2-one (Compound No. II.ae.040) as colourless crystals, m.p. 164 - 165 °C.
c) Preparation of 6-methyl-5-naphthalen-2-yl-4-(2,4,6-trifluorophenyl)-2H-pyridazin-3-one (Compound No. I.ae.196)
   A mixture of 5-hydroxy-5-methyl-4-naphthalen-2-yl-3-(2,4,6-trifluorophenyl)-5H-furan-2-one (Compound No. ll.ae.040, 2.8 g), 0.4 ml of hydrazine hydrate and 30 ml of 1-butanol is heated for 16 h to 120 °C. Subsequently, the mixture is cooled to 0 °C. The hereby obtained solid is filtered and washed with hexane to obtain 6-methyl-5-naphthalen-2-yl-4-(2,4,6-trifluorophenyl)-2H-pyridazin-3-one (Compound No. I.ae.196) as colourless crystals, m.p. 266-268 °C.
d) A mixture of 6-methyl-5-naphthalen-2-yl-4-(2,4,6-trifluorophenyl)-2H-pyridazin-3-one (Compound No. I.ae.196, 1.8 g) and 7.0 ml of phosphorus oxychloride are mixed and heated at 110 °C for 1 h. After cooling the reaction mixture is evaporated under reduced pressure. The remainder is taken up with ethyl acetate and water and the phases are separated. The organic layer is washed with water and brine, dried over sodium sulfate and evaporated under reduced pressure. The residue is purified by chromatography on silica gel, using a mixture of heptane / ethyl acetate 9 : 1 as eluent, to deliver 3-chloro-6-methyl-5-naphthaten-2-yl-4-(2,4,6-trifiuorophenyl)-pyndazine (Compound No.I.ae.198) as beige crystals (from diethyl ether / hexane), m.p. 86-89 °C.

### Example 2: This example illustrates the preparation of 3-methoxy-6-methyl-5-naphthalen-2-yl-4-(2,4,6-trifluorophenyl)-pyridazine (Compound No.I.ae.199) and 4-(2,6-difluoro-4-methoxyphenyl)-3-methoxy-6-methyl-5-naphthalen-2-yl-pyridazine (Compound No.l.ae.254)

A mixture of 3-chloro-6-methyl-5-naphthalen-2-y-4-(2,4,6-trifluorophenyl)-pyridazine (Compound No.l.ae.198, 0.9 g), sodium methoxide (30% solution in methanol, 0.7 g) and 10 ml of methanol is heated for 16 h to 60 °C. Subsequently the reaction mixture is cooled, diluted with water and extracted with ethyl acetate. The combined organic layer is washed with water and brine, dried over sodium sulfate and evaporated under reduced pressure. The remainder is purified by chromatography on silica gel, using a mixture of hexane / tert.butyl methyl ether 4 : 1 as eluent to obtain 3-methoxy-6-methyl-5-naphthalen-2-yl-4-(2,4,6-trifluorophenyl)-pyridazine (Compound I.ae.199) as colourless crystals, m.p. 138-140 °C and 4-(2,6-difluoro-4-methoxyphenyl)-3-methoxy-6-methyl-5-naphthalen-2-yl-pyridazine (Compound I.ae.254) as a foam.

### Example 3: This example illustrates the preparation of 3-fluoro-6-methyl-5-naphthalen-2-yl-4-(2,4,6-trifluorophenyl)-pyridazine (Compound No.l.ae.197)

A mixture of 3-chloro-6-methyl-5-naphthalen-2-yl-4-(2,4,6-trifluorophenyl)-pyridazine (Compound No. l.ae.198, 0.5 g), potassium fluoride (0.2 g) and 8 ml of dimethyl sulfoxide are mixed and heated to 140 °C for 72 h. Subsequently the reaction mixture is cooled, diluted with water and extracted with ethyl acetate. The combined organic layer is washed with water and brine, dried over sodium sulfate and evaporated under reduced pressure. The remainder is purified by chromatography on silica gel, using a mixture of hexane / ethyl acetate 9 : 1 as eluent to obtain 3-fluoro-6-methyl-5-naphthalen-2-yl-4-(2,4,6-trifluorophenyl)-pyridazine (Compound No.Lae.197) as colourless crystals (from diethyl ether / hexane), m.p. 108-110°C.

### Example 4: This example illustrates the preparation of 3,6-dimethyl-4-naphthalen-2-yl-5-(2,4,6-bVuoraphenyl)-pyridazine (Compound No.Lae.200)

1.5 ml of a 3 M solution of methylmagnesium bromide in diethyl ether is added slowly to a solution of 3-chloro-6-methyl-5-naphthalen-2-yl-4-(2,4,6-trifluorophenyl)-pyridazine (Compound No.l.ae.198, 0.3 g) and iron(III) acetylacetonate (0.03 g) in 10 ml of tetrahydrofuran and 1.5 ml of 1-methyl-2-pyrrolidinone (NMP) at room temperature. This mixture is stirred for 1.5 h at room temperature. The reaction is quenched with dilute HCl and the aqueous phase is extracted with tert-butyl methyl ether. The combined organic layer is dried over sodium sulfate and evaporated under reduced pressure. The residue is purified by chromatography on silica gel, using a mixture of hexane / ethyl acetate 3:1 as eluent to give 3,6 dimethyl-4-naphthalen-2-yl-5-(2,4,6-trifluorophenyl)-pyridazine (Compound No-Lae.200) as an oil.

Tables 1 and 2 below illustrate examples of individual compounds of formula I and formula II according to the invention.

**Table 1: individual compounds of formula I according to the invention**

| **Compound No.** | **R¹** | **R³** | **R⁴** |
|---|---|---|---|
| 001 | CH₃ | 2-fluorophenyl | OH |
| 002 | CH₃ | 2-fluorophenyl | F |
| 003 | CH₃ | 2-fluorophenyl | Cl |
| 004 | CH₃ | 2-fluorophenyl | OCH₃ |
| 005 | CH₃ | 2-tluorophenyl | CH₃ |
| 006 | CH₃ | 2-chlorophenyl | OH |
| 007 | CH₃ | 2-chlorophenyl | F |
| 008 | CH₃ | 2-chlorophenyl | Cl |
| 009 | CH₃ | 2-chlomphenyl | OCH₃ |
| 010 | CH₃ | 2-chlorophenyl | CH₃ |
| 011 | CH₃ | 2-trifluoromethylphenyl | OH |
| 012 | CH₃ | 2-trifluoromethylphenyl | F |
| 013 | CH₃ | 2-trifluoromethylphenyl | Cl |
| 014 | CH₃ | 2-trifluoromethylphenyl | OCH₃ |
| 015 | CH₃ | 2-trifluoromethylphenyl | CH₃ |
| 016 | CH₃ | 2-methylphenyl | OH |
| 017 | CH₃ | 2-methylphenyl | F |
| 018 | CH₃ | 2-methylphenyl | Cl |
| 019 | CH₃ | 2-methylphenyl | OCH₃ |
| 020 | CH₃ | 2-methylphenyl | CH₃ |
| 021 | CH₃ | 2,3-difluorophenyl | OH |
| 022 | CH₃ | 2,3-difluorophenyl | F |
| 023 | CH₃ | 2,3-difluorophenyl | Cl |
| 024 | CH₃ | 2,3-difluorophenyl | OCH₃ |
| 025 | CH₃ | 2,3-difluorophenyl | CH₃ |
| 026 | CH₃ | 2,4-difluorophenyl | OH |
| 027 | CH₃ | 2,4-difluorophenyl | F |
| 028 | CH₃ | 2,4-difluorophenyl | Cl |
| 029 | CH₃ | 2,4-difluorophenyl | OCH₃ |
| 030 | CH₃ | 2,4-difluorophenyl | CH₃ |
| 031 | CH₃ | 2,5-difluorophenyl | OH |
| 032 | CH₃ | 2,5-difluorophenyl | F |
| 033 | CH₃ | 2,5-difluorophenyl | Cl |
| 034 | CH₃ | 2,5-difluorophenyl | OCH₃ |
| 035 | CH₃ | 2,5-difluorophenyl | CH₃ |
| 036 | CH₃ | 2,6-difluorophenyl | OH |
| 037 | CH₃ | 2,6-difluorophenyl | F |
| 038 | CH₃ | 2,6-difluorophenyl | Cl |
| 039 | CH₃ | 2,6-difluorophenyl | OCH₃ |
| 040 | CH₃ | 2,6-difluorophenyl | CH₃ |
| 041 | CH₃ | 2,3-dichlorophenyl | OH |
| 042 | CH₃ | 2,3-dichlorophenyl | F |
| 043 | CH₃ | 2,3-dichlorophenyl | Cl |
| 044 | CH₃ | 2,3-dichlorophenyl | OCH₃ |
| 045 | CH₃ | 2,3-dichlorophenyl | CH₃ |
| 046 | CH₃ | 2,4-dichlorophenyl | OH |
| 047 | CH₃ | 2,4-dichlorophenyl | F |
| 048 | CH₃ | 2,4-dichlorophenyl | Cl |
| 049 | CH₃ | 2,4-dichlorophenyl | OCH₃ |
| 050 | CH₃ | 2,4-dichlorophenyl | CH₃ |
| 051 | CH₃ | 2,5-dichlorophenyl | OH |
| 052 | CH₃ | 2,5-dichlorophenyl | F |
| 053 | CH₃ | 2,5-dichlorophenyl | Cl |
| 054 | CH₃ | 2,5-dichlorophenyl | OCH₃ |
| 055 | CH₃ | 2,5-dichlorophenyl | CH₃ |
| 056 | CH₃ | 2,6-dichlorophenyt | OH |
| 057 | CH₃ | 2,6-dichlorophenyl | F |
| 058 | CH₃ | 2,6-dichlorophenyl | Cl |
| 059 | CH₃ | 2,6-dichlorophenyl | OCH₃ |
| 060 | CH₃ | 2,6-dichlorophenyl | CH₃ |
| 061 | CH₃ | 2-chloro-3-fluorophenyl | OH |
| 062 | CH₃ | 2-chloro-3-fluorophenyl | F |
| 063 | CH₃ | 2-chloro-3-filuorophenyl | Cl |
| 064 | CH₃ | 2-chloro-3-fluorophenyl | OCH₃ |
| 065 | CH₃ | 2-chloro-3-fluorophenyl | CH₃ |
| 066 | CH₃ | 2-chloro-4-fluorophenyl | OH |
| 067 | CH₃ | 2-chloro-4-fluorophenyl | F |
| 068 | CH₃ | 2-chloro-4-fluorophenyl | Cl |
| 069 | CH₃ | 2-chloro-4-fluorophenyl | OCH₃ |
| 070 | CH₃ | 2-chloro-4-fluorophenyl | CH₃ |
| 071 | CH₃ | 2-chloro-5-fluorophenyl | OH |
| 072 | CH₃ | 2-chloro-5-fluorophenyl | F |
| 073 | CH₃ | 2-chloro-5-fluorophenyl | Cl |
| 074 | CH₃ | 2-chloro-5-fluorophenyl | OCH₃ |
| 075 | CH₃ | 2-chloro-5-fluorophenyl | CH₃ |
| 076 . | CH₃ | 2-chloro-6-fluorophenyl | OH |
| 077 | CH₃ | 2-chloro-6-fluorophenyl | F |
| 078 | CH₃ | 2-chloro-6-fluorophenyl | Cl |
| 079 | CH₃ | 2-chloro-6-fluorophenyl | OCH₃ |
| 080 | CH₃ | 2-chloro-6-fluorophenyl | CH₃ |
| 081 | CH₃ | 3-chloro-2-fluomphenyt | OH |
| 082 | CH₃ | 3-chloro-2-fluoraphenyl | F |
| 083 | CH₃ | 3-chloro-2-fluorophenyl | Cl |
| 084 | CH₃ | 3-chloro-2-fluorophenyl | OCH₃ |
| 085 | CH₃ | 3-chloro-2-fluorophenyl | CH₃ |
| 086 | CH₃ | 4-chloro-2-fluorophenyl | OH |
| 087 | CH₃ | 4-chloro-2-fluorophenyl | F |
| 088 | CH₃ | 4-chloro-2-fluorophenyl | Cl |
| 089 | CH₃ | 4-chloro-2-fluorophenyl | OCH₃ |
| 090 | CH₃ | 4-chloro-2-fluorophenyl | CH₃ |
| 091 | CH₃ | 5-chloro-2-fluorophenyl | OH |
| 092 | CH₃ | 5-chloro-2-fluorophenyl | F |
| 093 | CH₃ | 5-chloro-2-fluorophenyl | Cl |
| 094 | CH₃ | 5-chloro-2-fluorophenyl | OCH₃ |
| 095 | CH₃ | 5-chloro-2-fluorophenyl | CH₃ |
| 096 | CH₃ | 2-fluoro-3-trifluoromethylphenyl | OH |
| 097 | CH₃ | 2-fluoro-3-trifluoromethylphenyl | F |
| 098 | CH₃ | 2-fluoro-3-trifluoromethylphenyl | Cl |
| 099 | CH₃ | 2-fluoro-3-trifluoromethylphenyl | OCH₃ |
| 100 | CH₃ | 2-fluoro-3-trifluoromethylphenyl | CH₃ |
| 101 | CH₃ | 2-fluoro-4-trifluoromethylphenyl | OH |
| 102 | CH₃ | 2-fluoro-4-trifluoromethylphenyl | F |
| 103 | CH₃ | 2-fluoro-4-trifluoromethylphenyl | Cl |
| 104 | CH₃ | 2-fluoro4-trifluommethylphenyl | OCH₃ |
| 105 | CH₃ | 2-fluoro-4-trifluoromethylphenyl | CH₃ |
| 106 | CH₃ | 2-fluoro-5-trifluoromethylphenyl | OH |
| 107 | CH₃ | 2-fluoro-5-trifluoromethylphenyl | F |
| 108 | CH₃ | 2-fluoro-5-trifluoromethylphenyl | Cl |
| 109 | CH₃ | 2-fluoro-5-trifluoromethylphenyl | OCH₃ |
| 110 | CH₃ | 2-fluoro-5-trifluoromethylphenyl | CH₃ |
| 111 | CH₃ | 2-fluoro-6-trifluoromethylphenyl | OH |
| 112 | CH₃ | 2-fluoro-6-trifluoromethylphenyl | F |
| 113 | CH₃ | 2-fluoro-6-trifluoromethylphenyl | Cl |
| 114 | CH₃ | 2-fluaro-6-trifluoromethylphenyl | OCH₃ |
| 115 | CH₃ | 2-fluoro-6-trifluoromethylphenyl | CH₃ |
| 116 | CH₃ | 2-chloro-3-trifluorometfiylphenyl | OH |
| 117 | CH₃ | 2-chloro-3-trifluoromethylphenyl | F |
| 118 | CH₃ | 2-chloro-3-trifluoromethylphenyl | Cl |
| 119 | CH₃ | 2-chloro-3-trifluoromethylphenyl | OCH₃ |
| 120 | CH₃ | 2-chloro-3-trifluoromehtylphenyl | CH₃ |
| 121 | CH₃ | 2-chloro-4-trifluoromethylphenyl | OH |
| 122 | CH₃ | 2-chloro-4-trifluoromethylphenyl | F |
| 123 | CH₃ | 2-chloro-4-trifluoromethylphenyl | Cl |
| 124 | CH₃ | 2-chloro-4-trifluoromethylphenyl | OCH₃ |
| 125 | CH₃ | 2-chloro-4-trifluoromethylphenyl | CH₃ |
| 126 | CH₃ | 2-chloro-5-trifluoromethylphenyl | OH |
| 127 | CH₃ | 2-chloro-5-trifluoromethylphenyl | F |
| 128 | CH₃ | 2-chloro-5-trifluoromethylphenyl | Cl |
| 129 | CH₃ | 2-chloro-5-trifluoromethylphenyl | OCH₃ |
| 130 | CH₃ | 2-chloro-5-trifluoromethylphenyl | CH₃ |
| 131 | CH₃ | 2-chloro-6-trifluoromethylphenyl | OH |
| 132 | CH₃ | 2-chloro-6-trifluoromethylphenyl | F |
| 133 | CH₃ | 2-chloro-6-trifluoromethylphenyl | Cl |
| 134 | CH₃ | 2-chloro-6-trifluoromethylphenyl | OCH₃ |
| 135 | CH₃ | 2-chloro-6-trifluoromethylphenyl | CH₃ |
| 136 | CH₃ | 4-fluoro-2-trifluoromethylphenyl | OH |
| 137 | CH₃ | 4-fluoro-2-trifluoromethylphenyl | F |
| 138 | CH₃ | 4-fluoro-2-trifluoromethylphenyl | Cl |
| 139 | CH₃ | 4-fluoro-2-trifluoromethylphenyl | OCH₃ |
| 140 | CH₃ | 4-fluoro-2-triffuoromethylphenyl | CH₃ |
| 141 | CH₃ | 4-chloro-2-trifluoromethylphenyl | OH |
| 142 | CH₃ | 4-chloro-2-trifluoromethylphenyl | F |
| 143 | CH₃ | 4-chloro-2-trifluoromethylphenyl | Cl |
| 144 | CH₃ | 4-chloro-2-trifluoromethylphenyl | OCH₃ |
| 145 | CH₃ | 4-chloro-2-trifluoromethylphenyl | CH₃ |
| 146 | CH₃ | 2-fluoro-3-methylphenyl | OH |
| 147 | CH₃ | 2-fluoro-3-methylphenyl | F |
| 148 | CH₃ | 2-fluoro-3-methylphenyl | Cl |
| 149 | CH₃ | 2-fluoro-3-methylphenyl | OCH₃ |
| 150 | CH₃ | 2-fluoro-3-methylphenyl | CH₃ |
| 151 | CH₃ | 2-fluoro-4-methylphenyl | OH |
| 152 | CH₃ | 2-fluoro-4-methylphenyl | F |
| 153 | CH₃ | 2-fluoro-4-methylphenyl | Cl |
| 154 | CH₃ | 2-fluoro-4-methylphenyl | OCH₃ |
| 155 | CH₃ | 2-fluoro-4-methylphenyl | CH₃ |
| 156 | CH₃ | 2-fluoro-5-methylphenyl | OH |
| 157 | CH₃ | 2-fluoro-5-methylphenyl | F |
| 158 | CH₃ | 2-fluoro-5-methylphenyl | Cl |
| 159 | CH₃ | 2-fluoro-5-methylphenyl | OCH₃ |
| 160 | CH₃ | 2-fluoro-5-methylphenyl | CH₃ |
| 161 | CH₃ | 2-fluoro-6-methylphenyl | OH |
| 162 | CH₃ | 2-fluoro-6-methylphenyl | F |
| 163 | CH₃ | 2-fluoro-6-methylphenyl | Cl |
| 164 | CH₃ | 2-fluoro-6-methylphenyl | OCH₃ |
| 165 | CH₃ | 2-fluoro-5-methylphenyl | CH₃ |
| 166 | CH₃ | 2-chloro-3-methylphonyl | OH |
| 167 | CH₃ | 2-chloro-3-methylphenyl | F |
| 168 | CH₃ | 2-chloro-3-methylphenyl | Cl |
| 169 | CH₃ | 2-chloro-3-methylphenyl | OCH₃ |
| 170 | CH₃ | 2-chloro-3-methylphenyl | CH₃ |
| 171 | CH₃ | 2-chloro-methylphenyl | OH |
| 172 | CH₃ | 2-chloro-4-methylphenyl | F |
| 173 | CH₃ | 2-chloro-4-methylphenyl | Cl |
| 174 | CH₃ | 2-chloro-4-methylphenyl | OCH₃ |
| 175 | CH₃ | 2-chloro-4-methylphenyl | CH₃ |
| 176 | CH₃ | 2-chloro-5-methylphenyl | OH |
| 177 | CH₃ | 2-chloro-5-methylphenyt | F |
| 178 | CH₃ | 2-chloro-5-methylphenyl | Cl |
| 179 | CH₃ | 2-chloro-5-methylphenyl | OCH₃ |
| 180 | CH₃ | 2-chloro-5-methylphenyl | CH₃ |
| 181 | CH₃ | 2-chloro-6-mothylphenyl | OH |
| 182 | CH₃ | 2-chloro-6-methylphenyl | F |
| 183 | CH₃ | 2-chloro-6-methylphenyl | Cl |
| 184 | CH₃ | 2-chloro-6-methylphenyl | OCH₃ |
| 185 | CH₃ | 2-chloro-6-methylphenyl | CH₃ |
| 186 | CH₃ | 4-fluoro-2-methylphenyl | OH |
| 187 | CH₃ | 4-fluoro-2-methylphenyl | F |
| 188 | CH₃ | 4-fluoro-2-methylphenyl | Cl |
| 189 | CH₃ | 4-fluoro-2-methylphenyl | OCH₃ |
| 190 | CH₃ | 4-fluoro-2-methylphenyl | CH₃ |
| 191 | CH₃ | 4-chloro-2-methylphenyl | OH |
| 192 | CH₃ | 4-chloro-2-methylphenyl | F |
| 193 | CH₃ | 4-chloro-2-mothylphenyl | Cl |
| 194 | CH₃ | 4-chloro-2-methylphenyl | OCH₃ |
| 195 | CH₃ | 4-chloro-2-methylphenyl | CH₃ |
| 196 | CH₃ | 2,4,6-trfluorophenyl | OH |
| 197 | CH₃ | 2,4,6-trifluorophenyl | F |
| 198 | CH₃ | 2,4,6-trifluorophenyl | Cl |
| 199 | CH₃ | 2,4,6-trifluorophenyl | OCH₃ |
| 200 | CH₃ | 2,4,6-trifluorophenyl | CH₃ |
| 201 | CH₃ | 2,3,6-trifluorophenyl | OH |
| 202 | CH₃ | 2,3,6-trifluorophenyl | F |
| 203 | CH₃ | 2,3,6-trifluorophenyl | Cl |
| 204 | CH₃ | 2,3,6-trifluorophenyl | OCH₃ |
| 205 | CH₃ | 2,3,6-trifluorophenyl | CH₃ |
| 206 | CH₃ | 2,3,4-trifluorophenyl | OH |
| 207 | CH₃ | 2,3,4-trifluorophenyl | F |
| 208 | CH₃ | 2,3,4-trifluorophenyl | Cl |
| 209 | CH₃ | 2,3,4-trffiuorophenyl | OCH₃ |
| 210 | CH₃ | 2,3,4-triftuorophenyl | CH₃ |
| 211 | CH₃ | 2,4,6-trichlorophenyl | OH |
| 212 | CH₃ | 2,4,6-trichlorophenyf | F |
| 213 | CH₃ | 2,4,6-trichlorophenyl | Cl |
| 214 | CH₃ | 2,4,6-trichlorophenyl | OCH₃ |
| 215 | CH₃ | 2,4,6-trichlorophenyl | CH₃ |
| 216 | CH₃ | 2,3,6-trichlorophenyl | OH |
| 217 | CH₃ | 2,3,6-trichlorophenyl | F |
| 218 | CH₃ | 2,3,6-trichlorophenyl | Cl |
| 219 | CH₃ | 2,3,6-trichlorophenyl | OCH₃ |
| 220 | CH₃ | 2,3,6-trichlorophenyl | CH₃ |
| 221 | CH₃ | 2,3,4-trichlorophenyl | OH |
| 222 | CH₃ | 2,3,4-trichlorophenyl | F |
| 223 | CH₃ | 2,3,4-trichlorophenyl | Cl |
| 224 | CH₃ | 2,3,4-trichlorophenyl | OCH₃ |
| 225 | CH₃ | 2,3,4-trichlorophenyl | CH₃ |
| 226 | CH₃ | 2,6-difluoro-4-methoxyphenyl | OH |
| 227 | CH₃ | 2,6-difluoro-4-methoxyphenyl | F |
| 228 | CH₃ | 2,6-difluoro-4-methoxyphenyl | Cl |
| 229 | CH₃ | 2,6-difluoro-4-methoxyphenyl | OCH₃ |
| 230 | CH₃ | 2,6-difluoro-4-methoxyphenyl | CH₃ |
| 231 | CH₃ | 2,6-difiuoro-4-trifluromethoxyphenyl | OH |
| 232 | CH₃ | 2,6-difluoro-4-trifluoromethoxyphenyl | F |
| 233 | CH₃ | 2,6-difluoro-4-trifluoromethoxyphenyl | Cl |
| 234 | CH₃ | 2,6-difluoro-4-trifluoromethoxyphenyl | OCH₃ |
| 235 | CH₃ | 2,6-difluoro-4-trifluoromethoxyphenyl | CH₃ |
| 236 | CH₃ | 2,6-difluoro-4-trifluoromethylphenyl | OH |
| 237 | CH₃ | 2,6-difluoro-4-trifluoromethylphenyl | F |
| 238 | CH₃ | 2,6-difluoro-4-difluoromethylphenyl | Cl |
| 239 | CH₃ | 2,6-difluoro-4-trifluoromethylphenyl | OCH₃ |
| 240 | CH₃ | 2,6-difluoro-4-trifluoromethylphenyl | CH₃ |
| 241 | CH₃ | 2,6-difluoro-4-cyanoyphenyl | OH |
| 242 | CH₃ | 2,6-difluoro-4-cyanoyphenyl | F |
| 243 | CH₃ | 2,6-difluoro-4-cyanophenyl | Cl |
| 244 | CH₃ | 2,6-difluoro-4-cyanophenyl | OCH₃ |
| 245 | CH₃ | 2,6-difluoro-4-cyanophenyl | CH₃ |
| 246 | CH₃ | 2,6-difluoro-4-methylphenyl | OH |
| 247 | CH₃ | 2,6-difluoro-4-methylphenyl | F |
| 248 | CH₃ | 2,6-difluoro-4-methylphenyl | Cl |
| 249 | CH₃ | 2,6-difluoro-4-methylphenyl | OCH₃ |
| 250 | CH₃ | 2,6-difluoro-4-methylphenyl | CH₃ |
| 251 | CH₃ | 2,6-dichloro-4-methoxyphenyl | OH |
| 252 | CH₃ | 2,6-dichloro-4-methoxyphenyl | F |
| 253 | CH₃ | 2,6-dichloro-4-methoxyphenyl | Cl |
| 254 | CH₃ | 2,6-dichloro-4-methoxyphenyl | OCH₃ |
| 255 | CH₃ | 2,6-dichloro-4-methoxyphenyl | CH₃ |
| 256 | CH₃ | 2,6-dichloro-4-trifluoromethoxyphenyl | OH |
| 257 | CH₃ | 2,6-dichloro-4-trifluoromethoxyphenyl | F |
| 258 | CH₃ | 2,6-dichloro-4-trifluoromethoxyphenyl | Cl |
| 259 | CH₃ | 2,6-dichloro-4-trifluoromethoxyphenyl | OCH₃ |
| 260 | CH₃ | 2,6-dichloro-4-trifluoromethoxyphenyl | CH₃ |
| 261 | CH₃ | 2,6-dichloro-4-trifluoromethylphenyl | OH |
| 262 | CH₃ | 2,6-dichloro-4-trifluoromethylphenyl | F |
| 263 | CH₃ | 2,6-dichloro-4-trifluoromethylphenyl | Cl |
| 264 | CH₃ | 2,6-dichloro-4-trifluoromethylphenyl | OCH₃ |
| 265 | CH₃ | 2,6-dichloro-4-tTifluoromethylphenyt | CH₃ |
| 266 | CH₃ | 2,6-dichloro-4-cyanophenyl | OH |
| 267 | CH₃ | 2,6-dichloro-4-cyanophenyl | F |
| 268 | CH₃ | 2,6-dichloro-4-cyanophenyl | Cl |
| 269 | CH₃ | 2,6-dichloro-4-cyanophenyl | OCH₃ |
| 270 | CH₃ | 2,6-dichloro-4-cyanophenyl | CH₃ |
| 271 | CH₃ | 2,6-dichloro-4-methylphenyl | OH |
| 272 | CH₃ | 2,6-dichloro-4-methylphenyl | F |
| 273 | CH₃ | 2,6-dichloro-4-methylphenyl | Cl |
| 274 | CH₃ | 2,6-dichloro-4-methylphenyl | OCH₃ |
| 275 | CH₃ | 2,6-dichloro-4-methylphenyl | CH₃ |
| 276 | CH₃ | pentafluorophenyl | OH |
| 277 | CH₃ | pentafluorophenyl | F |
| 278 | CH₃ | pentafluorophenyl | Cl |
| 279 | CH₃ | pentafluorophenyl | OCH₃ |
| 280 | CH₃ | pentafluorophenyl | CH₃ |

where
a) 280 compounds of formula (I.a): 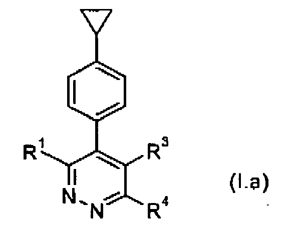 wherein R¹, R³ and R⁴ are as defined in Table 1.
b) 280 compounds of formula (I.b): 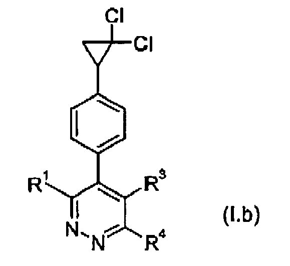 wherein R¹, R³ and R⁴ are as defined in Table 1.
c) 280 compounds of formula (I.c): 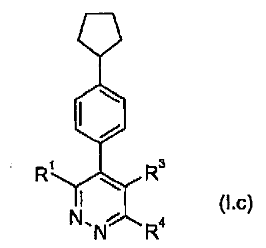 wherein R¹, R³ and R⁴ are as defined in Table 1.
d) 280 compounds of formula (I.d): 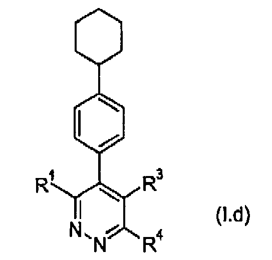 wherein R¹, R³ and R⁴ are as defined in Table 1.
e) 280 compounds of formula (I.e): 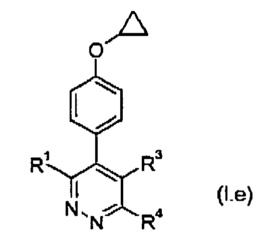 wherein R¹, R³ and R⁴ are as defined in Table 1.
f) 280 compounds of formula (I.f): 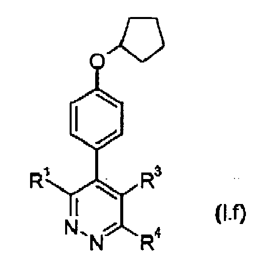 wherein R¹, R³ and R⁴ are as defined in Table 1.
g) 280 compounds of formula (I.g): 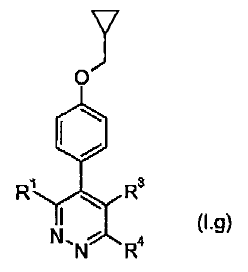 wherein R¹, R³ and R⁴ are as defined in Table 1.
h) 280 compounds of formula (I.h): 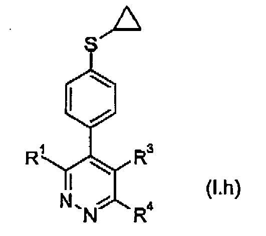 wherein R¹, R³ and R⁴ are as defined in Table 1.
i) 280 compounds of formula (I.i): 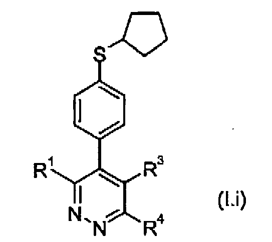 wherein R¹, R³ and R⁴ are as defined in Table 1.
j) 280 compounds of formula (I.j): 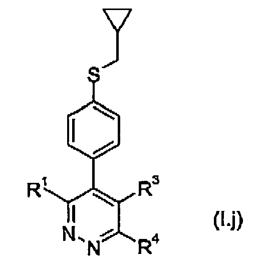 wherein R¹, R³ and R⁴ are as defined in Table 1.
k) 280 compounds of formula (l.k): 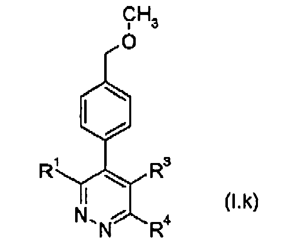 wherein R¹, R³ and R⁴ are as defined in Table 1.
l) 280 compounds of formula (l.l): 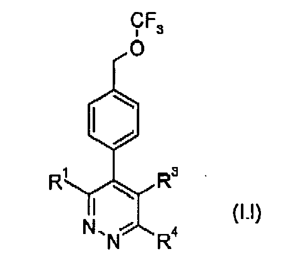 wherein R¹, R³ and R⁴ are as defined in Table 1.
m) 280 compounds of formula (l.m): 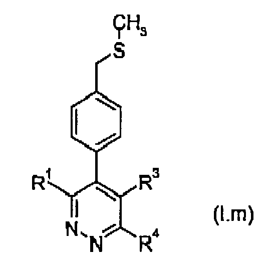 wherein R¹, R³ and R⁴ are as defined in Table 1.
n) 280 compounds of formula (I.n): 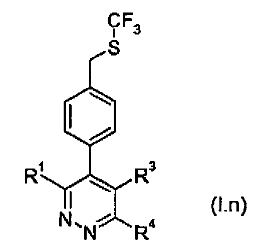 wherein R¹, R³ and R⁴ are as defined in Table 1.
o) 280 compounds of formula (I.o): 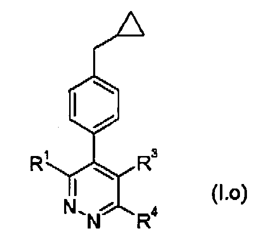 wherein R¹, R³ and R⁴ are as defined in Table 1.
p) 280 compounds of formula (I.p): 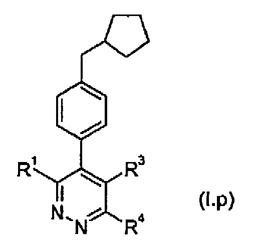 wherein R¹, R³ and R⁴ are as defined in Table 1.
q) 280 compounds of formula (I.q): 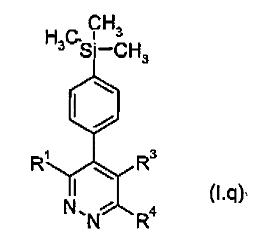 wherein R¹, R³ and R⁴ are as defined in Table 1.
r) 280 compounds of formula (Lr): 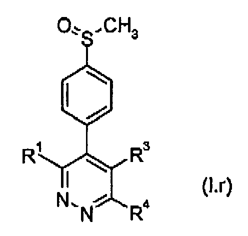 wherein R¹, R³ and R⁴ are as defined in Table 1.
s) 280 compounds of formula (I.s): 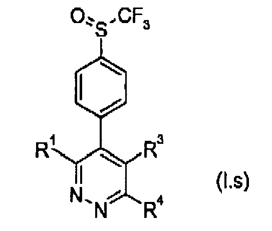 wherein R¹, R³ and R⁴ are as defined in Table 1.
t) 280 compounds of formula (I.t): 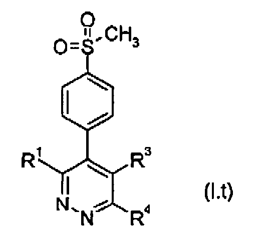 wherein R¹, R³ and R⁴ are as defined in Table 1.
u) 280 compounds of formula (I.u): 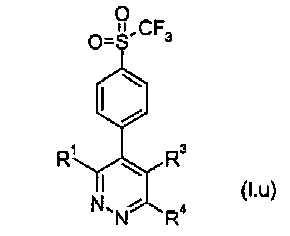 wherein R¹, R³ and R⁴ are as defined in Table 1.
v) 280 compounds of formula (I.v): 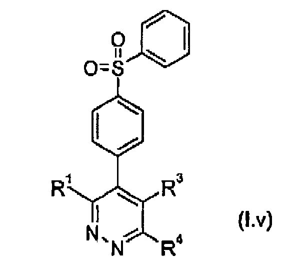 wherein R¹, R³ and R⁴ are as defined in Table 1.
w) 280 compounds of formula (I.w): 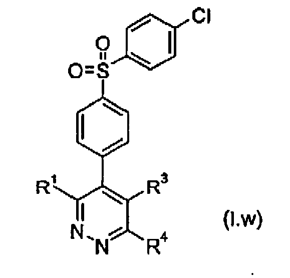 wherein R¹, R³ and R⁴ are as defined in Table 1.
x) 280 compounds of formula (I.x): 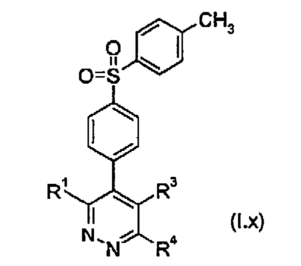 wherein R¹, R³ and R⁴are as defined in Table 1.
y) 280 compounds of formula (I.y): 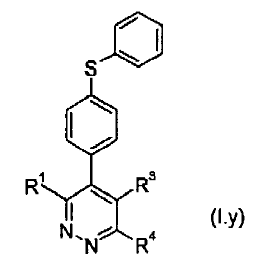 wherein R¹, R³ and R⁴are as defined in Table 1.
z) 280 compounds of formula (I.z): 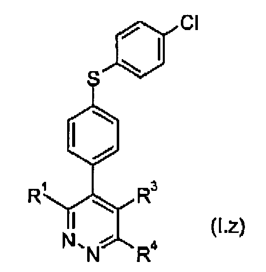 wherein R¹, R³ and R⁴ are as defined in Table 1.

aa) 280 compounds of formula (I.aa): 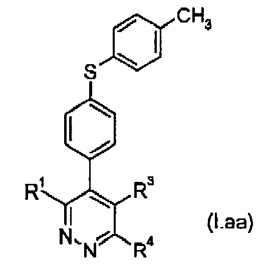 wherein R¹, R³ and R⁴ are as defined in Table 1.
ab) 280 compounds of formula (I.ab): 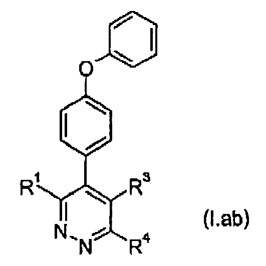 wherein R¹, R³ and R⁴ are as defined in Table 1.
ac) 280 compounds of formula (I.ac): 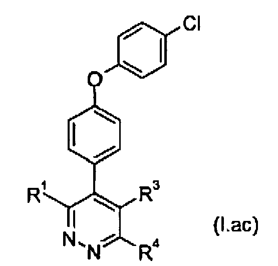 wherein R¹, R³ and R⁴ are as defined in Table 1.
ad) 280 compounds of formula (I.ad): 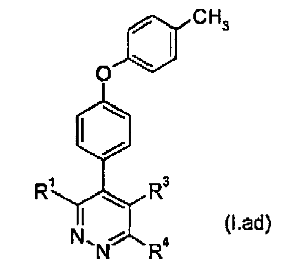 wherein R¹, R³ and R⁴ are as defined in Table 1.
ae) 280 compounds of formula (I.ae): 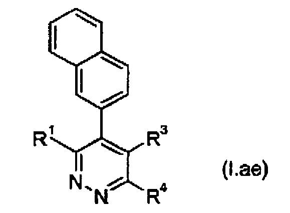 wherein R¹, R³ and R⁴ are as defined in Table 1.
af) 280 compounds of formula (I.af): 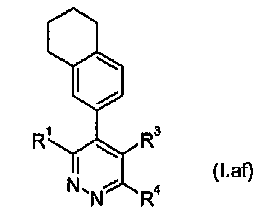 wherein R¹, R³ and R⁴ are as defined in Table 1.
ag) 280 compounds of formula (I.ag): 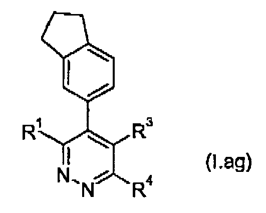 wherein R¹, R³ and R⁴ are as defined in Table 1.
ah) 280 compounds of formula (I.ah): 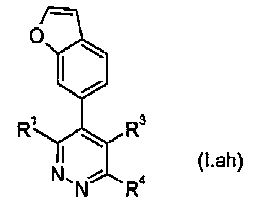 wherein R¹, R³ and R⁴ are as defined in Table 1.
ai) 280 compounds of formula (I.ai): 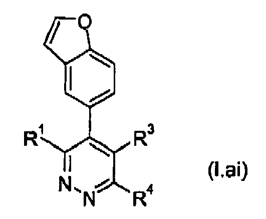 wherein R¹, R³ and R⁴ are as defined in Table 1.
aj) 280 compounds of formula (I.aj): 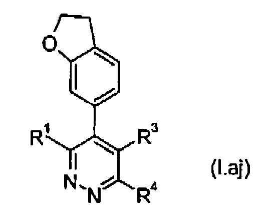 wherein R¹, R³ and R⁴ are as defined in Table 1.
ak) 280 compounds of formula (I.ak): 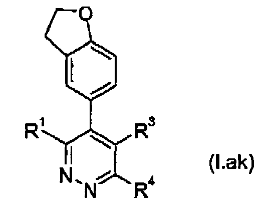 wherein R¹, R³ and R⁴ are as defined in Table 1.
al) 280 compounds of formula (I.al): 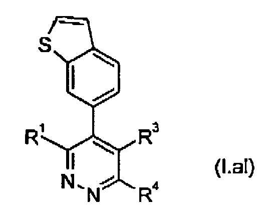 wherein R¹, R³ and R⁴ are as defined in Table 1.
am) 280 compounds of formula (I.am): 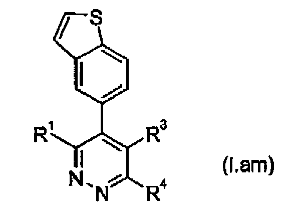 wherein R¹, R³ and R⁴ are as defined in Table 1.
an) 280 compounds of formula (I.an): 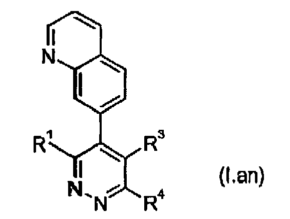 wherein R¹, R³ and R⁴ are as defined in Table 1.
ao) 280 compounds of formula (I.ao): 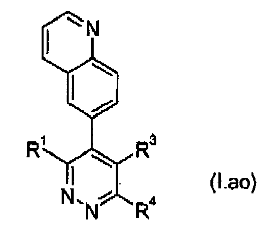 wherein R¹, R³ and R⁴ are as defined in Table 1.
ap) 280 compounds of formula (I.ap): 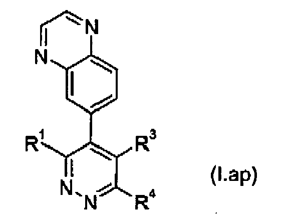 wherein R¹, R³ and R⁴ are as defined in Table 1.
aq) 280 compounds of formula (I.aq): 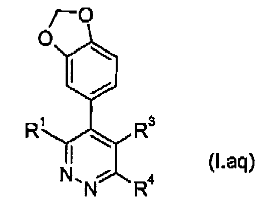 wherein R¹, R³ and R⁴ are as defined in Table 1.
ar) 280 compounds of formula (I.ar): 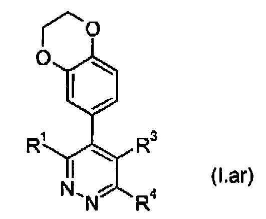 wherein R¹, R³ and R⁴ are as defined in Table 1.
as) 280 compounds of formula (I.as): 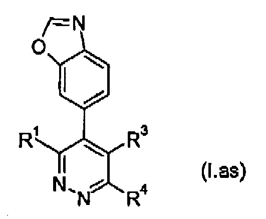 wherein R¹, R³ and R⁴ are as defined in Table 1.
at) 280 compounds of formula (I.at): 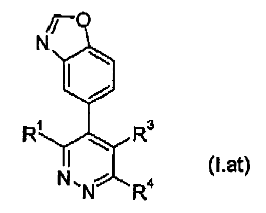 wherein R¹, R³ and R⁴ are as defined in Table 1.
au) 280 compounds of formula (I.au): 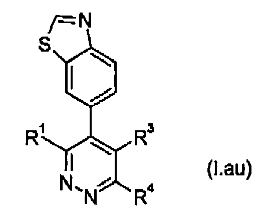 wherein R¹, R³ and R⁴ are as defined in Table 1.
av) 280 compounds of formula (I.av): 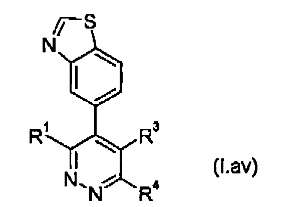 wherein R¹, R³ and R⁴ are as defined in Table 1.
aw) 280 compounds of formula (I.aw): 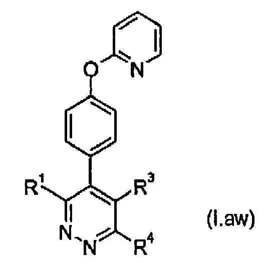 wherein R¹, R³ and R⁴are as defined in Table 1.
ax) 280 compounds of formula (I.ax): 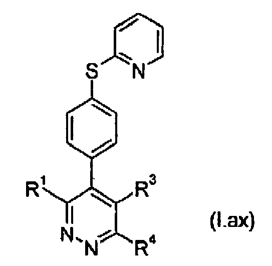 wherein R¹, R³ and R⁴ are as defined in Table 1.
ay) 280 compounds of formula (I.ay): 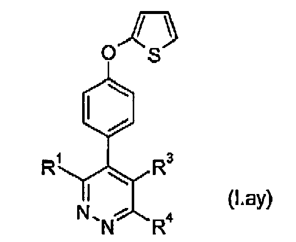 wherein R¹, R³ and R⁴are as defined in Table 1.
az) 280 compounds of formula (I.az): 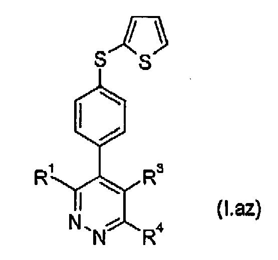 wherein R¹, R³ and R⁴ are as defined in Table 1.

**Table 2: individual compounds of formula II according to the invention**

| **Compound No.** | **R¹** | **R³** |
|---|---|---|
| 001 | CH₃ | 2-fluorophenyl |
| 002 | CH₃ | 2-chlorophenyl |
| 003 | CH₃ | 2-trifluoromethylphenyl |
| 004 | CH₃ | 2-methylphenyl |
| 005 | CH₃ | 2,3-difluorophenyl |
| 006 | CH₃ | 2,4-difluorophenyl |
| 007 | CH₃ | 2,5-difluorophenyl |
| 008 | CH₃ | 2,6-difluorophenyl |
| 009 | C_{H3} | 2,3-dichlorophenyl |
| 010 | CH₃ | 2,4-dichlorophenyl |
| 011 | CH₃ | 2,5-dichlorophenyl |
| 012 | CH₃ | 2,6-dichlorophenyl |
| 013 | CH₃ | 2-chloro-3-fluorophenyl |
| 014 | CH₃ | 2-chloro-4-fluorophenyl |
| 015 | CH₃ | 2-chloro-5-fluorophenyl |
| 016 | CH₃ | 2-chloro-6-fluorophenyl |
| 017 | CH₃ | 3-chloro-2-fluorophenyl |
| 018 | CH₃ | 4-chloro-2-fluorophenyl |
| 019 | CH₃ | 5-chloro-2-fluorophenyl |
| 020 | CH₃ | 2-fluoro-3-trifluoromethylphenyl |
| 021 | CH₃ | 2-fluoro-4-trifluoromethylphenyl |
| 022 | CH₃ | 2-fluoro-5-trifluoromethylphenyl |
| 023 | CH₃ | 2-fluoro-6-trifluoromethylphenyl |
| 024 | CH₃ | 2-chloro-3-trifluoromethylphenyl |
| 025 | CH₃ | 2-chloro-4-trifluoromethylphenyl |
| 026 | CH₃ | 2-chloro-5-trifluoromethylphenyl |
| 027 | CH₃ | 2-chloro-6-trifluommethylphenyl |
| 028 | CH₃ | 4-fluoro-2-trifluoromethylphenyl |
| 029 | CH₃ | 4-chloro-2-trifluoromethylphenyl |
| 030 | CH₃ | 2-fluoro-3-mothylphenyl |
| 031 | CH₃ | 2-fluoro-4-methylphenyl |
| 032 | CH₃ | 2-fluoro-5-methylphenyl |
| 033 | CH₃ | 2-fluoro-6-methylphenyl |
| 034 | CH₃ | 2-chloro-3-methylphenyl |
| 035 | CH₃ | 2-chloro-4-methylphenyl |
| 036 | CH₃ | 2-chloro-5-methylphenyl |
| 037 | CH₃ | 2-chloro-6-methylphenyl |
| 038 | CH₃ | 4-fluoro-2-methylphenyl |
| 039 | CH₃ | 4-chloro-2-methylphenyl |
| 040 | CH₃ | 2,4,6-trifluorophenyl |
| 041 | CH₃ | 2,3,6-trifluorophenyl |
| 042 | CH₃ | 2,3,4-trifluorophenyl |
| 043 | CH₃ | 2,4,6-trichlorophenyl |
| 044 | CH₃ | 2,3,6-trichlorophenyl |
| 045 | CH₃ | 2,3,4-trichlorophenyl |
| 046 | CH₃ | 2,6-difluoro-4-methoxyphenyl |
| 047 | CH₃ | 2,6-difluoro-4-trifluoromethoxyphenyl |
| 048 | CH₃ | 2,6-difluoro-4-trifluoromethylphenyl |
| 049 | CH₃ | 2,6-difluoro-4-cyanoypheny |
| 050 | CH₃ | 2,6-difluoro-4-methylphenyl |
| 051 | CH₃ | 2,6-dichloro-4-methoxyphenyl |
| 052 | CH₃ | 2,6-dichloro-4-trifluoromethoxyphenyl |
| 053 | CH₃ | 2,6-dichloro-4-trifluoromethylphenyl |
| 054 | CH₃ | 2,6-dirhloro-4-cyanophenyl |
| 055 | CH₃ | 2,6-dichloro-4-methylphenyl |
| 056 | CH₃ | pentafluorophenyl |

where
a) 56 compounds of formula (II.a): 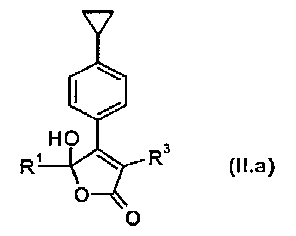 wherein R¹ and R³ are as defined in Table 2.
b) 56 compounds of formula (II.b): 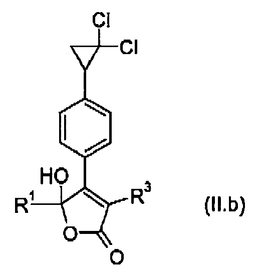 wherein R¹ and R³ are as defined in Table 2.
c) 56 compounds of formula (II.c): 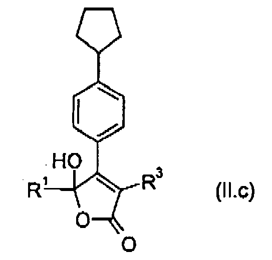 wherein R¹ and R³ are as defined in Table 2.
d) 56 compounds of formula (II.d): 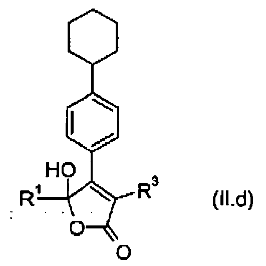 wherein R¹ and R³ are as defined in Table 2.
e) 56 compounds of formula (II.e): 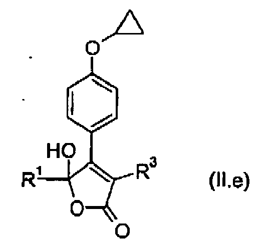 wherein R¹ and R³ are as defined in Table 2.
f) 56 compounds of formula (II.f): 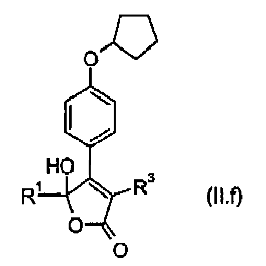 wherein R¹ and R³ are as defined in Table 2.
g) 56 compounds of formula (II.g): 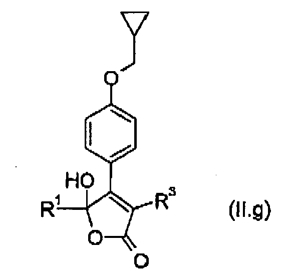 wherein R¹ and R³ are as defined in Table 2.
h) 56 compounds of formula (II.h): 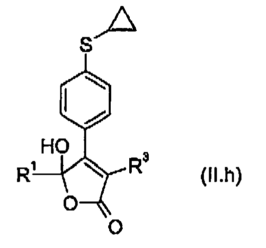 wherein R¹ and R³ are as defined in Table 2.
i) 56 compounds of formula (II.i): 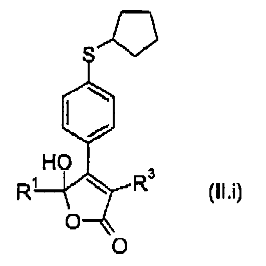 wherein R¹ and R³ are as defined in Table 2.
j) 56 compounds of formula (II.j): 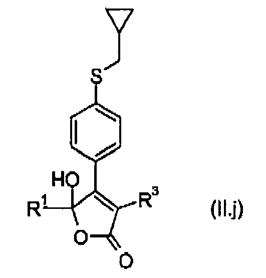 wherein R¹ and R³ are as defined in Table 2.
k) 56 compounds of formula (II.k): 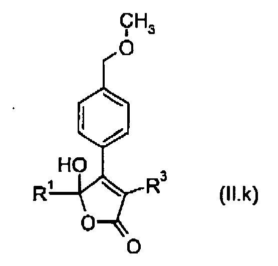 wherein R¹ and R³ are as defined in Table 2.
l) 56 compounds of formula (II.I): 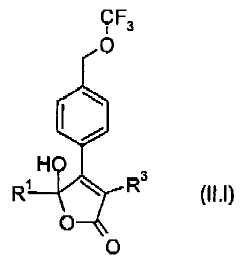 wherein R¹ and R³ are as defined in Table 2.
m) 56 compounds of formula (II.m): 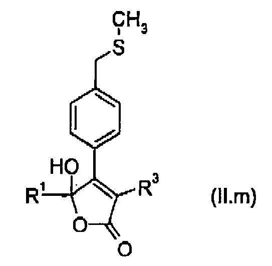 wherein R¹ and R³ are as defined in Table 2.
n) 56 compounds of formula (II.n): 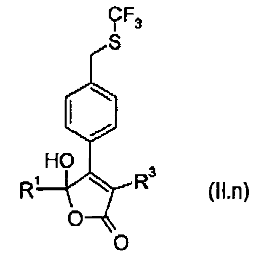 wherein R¹ and R³ are as defined in Table 2.
o) 56 compounds of formula (II.o): 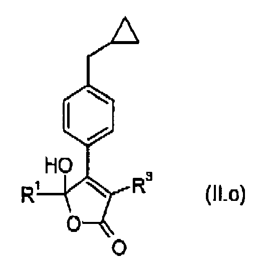 wherein R¹ and R³ are as defined in Table 2.
p) 56 compounds of formula (II.p): 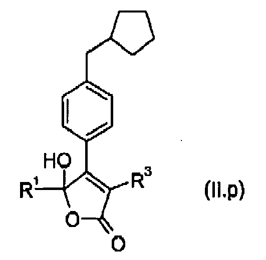 wherein R¹ and R³ are as defined in Table 2.
q) 56 compounds of formula (II.q): 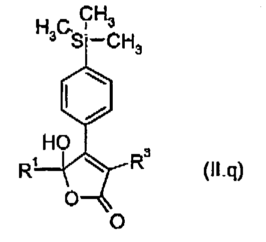 wherein R¹ and R³ are as defined in Table 2.
r) 56 compounds of formula (II.r): 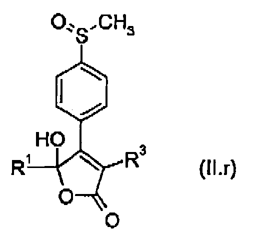 wherein R¹ and R³ are as defined in Table 2.
s) 56 compounds of formula (II.s): 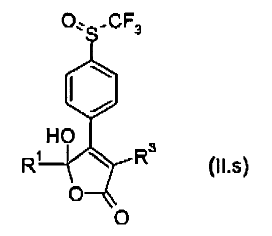 wherein R¹ and R³ are as defined in Table 2.
t) 56 compounds of formula (II.t): 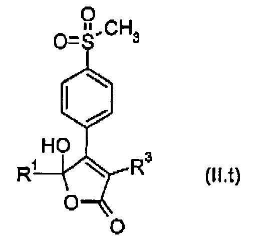 wherein R¹ and R³ are as defined in Table 2.
u) 56 compounds of formula (II.u): 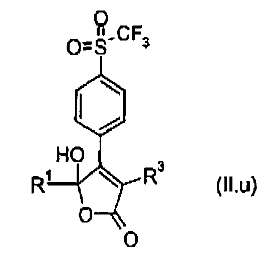 wherein R¹ and R³ are as defined in Table 2.
v) 56 compounds of formula (II.v): 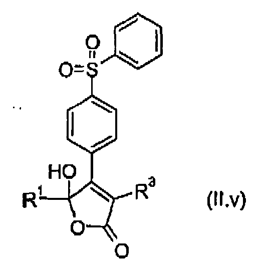 wherein R¹ and R³ are as defined in Table 2.
w) 56 compounds of formula (II.w): 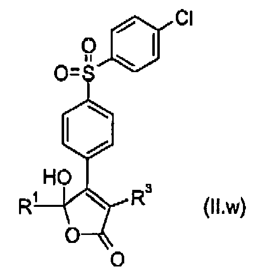 wherein R¹ and R³ are as defined in Table 2.
x) 56 compounds of formula (II.x): 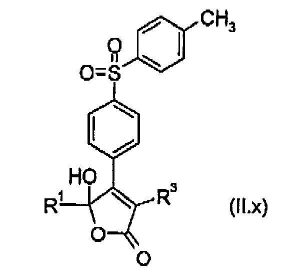 wherein R¹ and R³ are as defined in Table 2.
y) 56 compounds of formula (II.y): 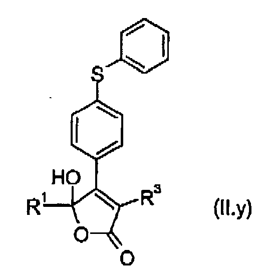 wherein R¹ and R³ are as defined in Table 2.
z) 56 compounds of formula (II.z): 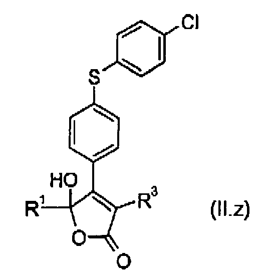 wherein R¹ and R³ are as defined in Table 2.

aa) 56 compounds of formula (II.aa): 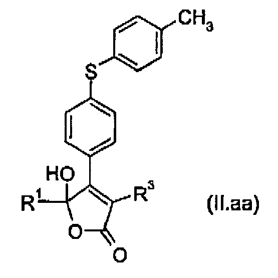 wherein R¹ and R³ are as defined in Table 2.
ab) 56 compounds of formula (II.ab): 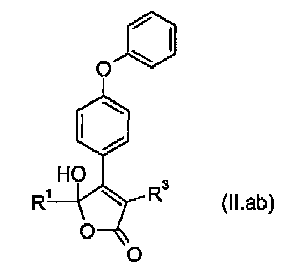 wherein R¹ and R³ are as defined in Table 2.
ac) 56 compounds of formula (II.ac). 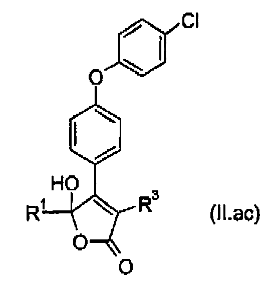 wherein R¹ and R³ are as defined in Table 2.
ad) 56 compounds of formula (II.ad): 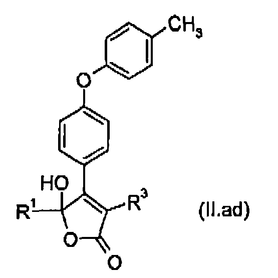 wherein R¹ and R³ are as defined in Table 2.
ae) 56 compounds of formula (II.ae): 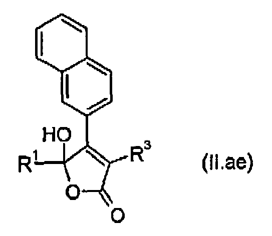 wherein R¹ and R³ are as defined in Table 2.
af) 56 compounds of formula (II.af): 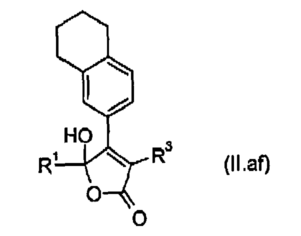 wherein R¹ and R³ are as defined in Table 2.
ag) 56 compounds of formula (II.ag): 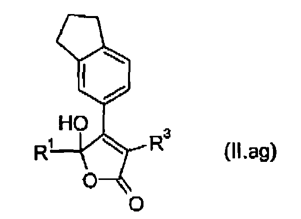 wherein R¹ and R³ are as defined in Table 2.
ah) 56 compounds of formula (II.ah): 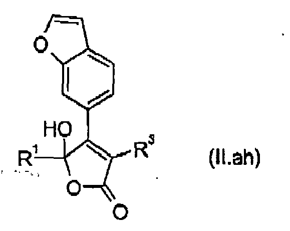 wherein R¹ and R³ are as defined in Table 2.
ai) 56 compounds of formula (II.ai): 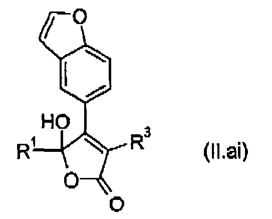 wherein R¹ and R³ are as defined in Table 2.
aj) 56 compounds of formula (II.aj): 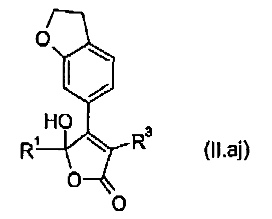 wherein R¹ and R³ are as defined in Table 2.
ak) 56 compounds of formula (II.ak): 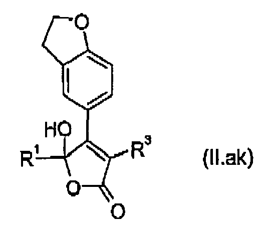 wherein R¹ and R³ are as defined in Table 2.
al) 56 compounds of formula (II.al): 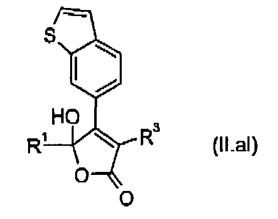 wherein R¹ and R³ are as defined in Table 2.
am) 56 compounds of formula (II.am): 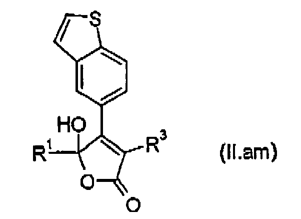 wherein R¹ and R³ are as defined in Table 2.
an) 56 compounds of formula (II.an): 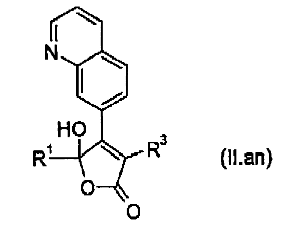 wherein R¹ and R³ are as defined in Table 2.
ao) 56 compounds of formula (II.ao): 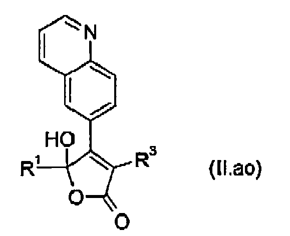 wherein R¹ and R³ are as defined in Table 2.
ap) 56 compounds of formula (II.ap): 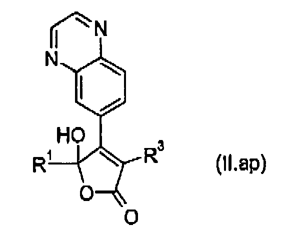 wherein R¹ and R³ are as defined in Table 2.
aq) 56 compounds of formula (II.aq): 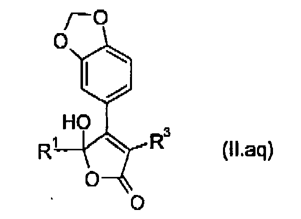 wherein R¹ and R³ are as defined in Table 2.
ar) 56 compounds of formula (II.ar): 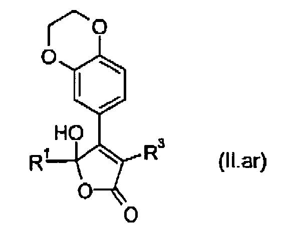 wherein R¹ and R³ are as defined in Table 2.
as) 56 compounds of formula (II.as): 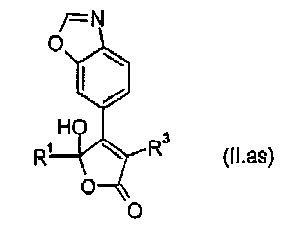 wherein R¹ and R³ are as defined in Table 2.
at) 56 compounds of formula (II.at): 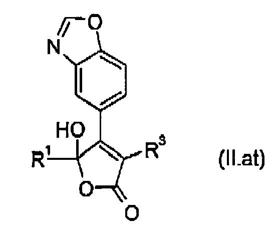 wherein R¹ and R³ are as defined in Table 2.
au) 56 compounds of formula (II.au): 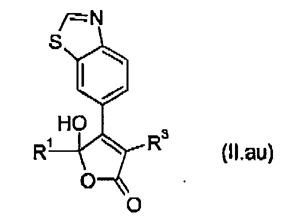 wherein R¹ and R³ are as defined in Table 2.
av) 56 compounds of formula (II.av): 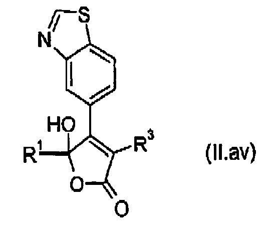 wherein R¹ and R³ are as defined in Table 2.
aw) 56 compounds of formula (II.aw): 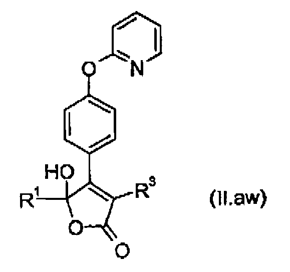 wherein R¹ and R³ are as defined in Table 2.
ax) 56 compounds of formula (II.ax): 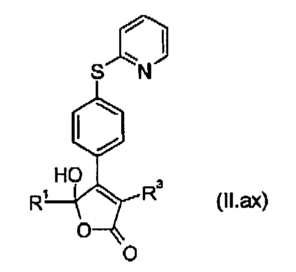 wherein R¹ and R³ are as defined in Table 2.
ay) 56 compounds of formula (II.ay): 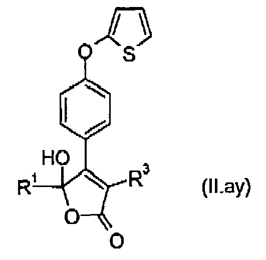 wherein R¹ and R³ are as defined in Table 2.
az) 56 compounds of formula (II.az): 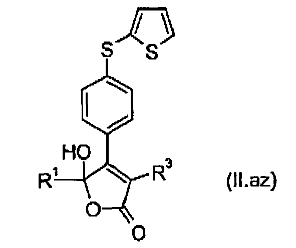 wherein R¹ and R³ are as defined in Table 2.

Throughout this description, temperatures are given in degrees Celsius; "NMR" means nuclear magnetic resonance spectrum; and "%" is percent by weight, unless corresponding concentrations are indicated in other units.

The following abbreviations are used throughout this description:

| | | | |
|---|---|---|---|
| m.p. = | melting point | br = | broad |
| s = | singlet | dd = | doublet of doublets |
| d = | doublet | dt = | doublet of triplets |
| t = | triplet | q = | quartet |
| m = | multiplet | ppm = | parts per million |

Table 3 shows selected melting point and selected NMR data, all with CDCl₃ as the solvent (unless otherwise stated, no attempt is made to list all characterising data in all cases) for compounds of Tables 1 and 2.

**Table 3: Melting point and selected NMR data for compounds of Tables 1 and 2**

| Compound Number | ¹H-NMR data (ppm/multiplicity/number of Hs) | m.p. (°C) |
|---|---|---|
| I.t.196 | | 297 - 298 |
| I.t.198 | | 210 - 211 |
| I.ac.196 | | 224 - 226 |
| I.ac.197 | | 109 - 112 |
| I.ac.199 | | 124 - 127 |
| I.ae.196 | | 266 - 268 |
| I.ae.197 | | 108 - 110 |
| I.ae.198 | | 86 - 89 |
| I.ae.199 | | 138 - 140 |
| II.t.040 | | 220 - 222 |
| II.ae.040 | | 164 - 165 |

The compounds according to the present invention can be prepared according to the above-mentioned reaction schemes, in which, unless otherwise stated, the definition of each variable is as defined above for a compound of formula (I).

### Biological examples

### Alternaria solani / tomato / preventive (Action against Alternaria on tomato)

4 weeks old tomato plants cv. Roter Gnom are treated with the formulated test compound in a spray chamber. Two days after application tomato plants are inoculated by spraying a spore suspension on the test plants. After an incubation period of 4 days at 22 °C / 18 °C and 95% r. h. in a greenhouse the disease incidence is assessed.

Compounds of formula I according to the invention, in particular compounds I.t.198, I.ac.197, I.ae.198 and l.ae.1 99 at 200 ppm inhibit fungal infestation in this test to at least 80 %, while under the same conditions untreated control plants are infected by the phytopathogenic fungi to over 80 %.

### Botrytis cinerea / tomato / preventive (Action against Botrytis on tomato)

4 weeks old tomato plants cv. Roter Gnom are treated with the formulated test compound in a spray chamber. Two days after application tomato plants are inoculated by spraying a spore suspension on the test plants. After an incubation period of 3 days at 20 °C and 95% r. h. in a greenhouse the disease incidence is assessed.

Compounds of formula I according to the invention, in particular compounds I.ac.197, I.ac.199, I. ae.197, I.ae.198 and I.ae.199 at 200 ppm inhibit fungal infestation in this test to at least 80 %, while under the same conditions untreated control plants are infected by the phytopathogenic fungi to over 80 %.

### Puccinia recondita / wheat / preventive (Action against brown rust on wheat)

1 week old wheat plants cv. Arina are treated with the formulated test compound in a spray chamber. One day after application wheat plants are inoculated by spraying a spore suspension (1 x 105 uredospores/ml) on the test plants. After an incubation period of 1 day at 20 °C and 95% r. h. plants are kept for 10 days 20 °C /18 °C (day/night) and 60% r.h. in a greenhouse. The disease incidence is assessed 11 days after inoculation.

Compounds of formula I according to the invention, in particular compounds I.ac.197, I. ae.197 and I.ae. 198 at 200 ppm inhibits fungal infestation in this test to at least 80 %, while under the same conditions untreated control plants are infected by the phytopathogenic fungi to over 80 %.

### Magnaporthe grisea (Pyricularia oryzae) / rice / preventive (Action against rice blast)

3 weeks old rice plants cv. Koshihikari are treated with the formulated test compound in a spray chamber. Two days after application rice plants are inoculated by spraying a spore suspension (1 x 10⁵ conidia/ml) on the test plants. After an incubation period of 6 days at 25°C and 95% r. h. the disease incidence is assessed.

Compounds of formula I according to the invention, in particular compounds l.t.198, l.ac.199, l. ae.197 and l. ae.198 at 200 ppm inhibit fungal infestation in this test to at least 80 %, while under the same conditions untreated control plants are infected by the phytopathogenic fungi to over 80 %.

### Pyrenophora teres (Helminthosporium teres) / barley / Preventive (Action against net blotch on barley)

1-week-old barley plants cv. Regina are treated with the formulated test compound in a spray chamber. Two days after application barley plants are inoculated by spraying a spore suspension (2.6 x 10⁴ conidia/ml) on the test plants. After an incubation period of 4 days at 20 °C and 95% r. h. the disease incidence is assessed.

Compounds of formula I according to the invention, in particular compounds I.t.198, I.ac.197, I. ae.197, I.ae.198 and I.ae.199 at 200 ppm inhibit fungal infestation in this test to at least 80 %, while under the same conditions untreated control plants are infected by the phytopathogenic fungi to over 80 %.

### Septoria tritici / wheat / preventive (Action against Septoria leaf spot on wheat)

2 weeks old wheat plants cv. Riband are treated with the formulated test compound in a spray chamber. One day after application wheat plants are inoculated by spraying a spore suspension (10⁶ conidia/ml) on the test plants. After an incubation period of 1 day at 22 °C / 21 °C and 95% r. h. plants are kept at 22 °C / 21 °C and 70% r.h. in a greenhouse. The disease incidence is assessed 16 - 18 days after inoculation.

Compounds of formula I according to the invention, in particular compounds l.ac. 197, I.ac.199, I. ae.197 and I.ae. 198 at 200 ppm inhibits fungal infestation in this test to at least 80 %, while under the same conditions untreated control plants are infected by the phytopathogenic fungi to over 80 %.

### Uncinula necator / grape / preventive (Action against powdery mildew on grape)

5 weeks old grape seedlings cv. Gutedel are treated with the formulated test compound in a spray chamber. One day after application grape plants are inoculated by shaking plants infected with grape powdery mildew above the test plants. After an incubation period of 7 days at 24 °C /22 °C and 70% r. h. under a light regime of 14/10 h (light/dark) the disease incidence is assessed.

Compounds of formula I according to the invention, in particular compounds l.ac.197, l.ac.199, I. ae. 198 and l.ae.199 at 200 ppm inhibit fungal infestation in this test to at least 80 %, while under the same conditions untreated control plants are infected by the phytopathogenic fungi to over 80 %.

## Claims

1. A compound of formula I: 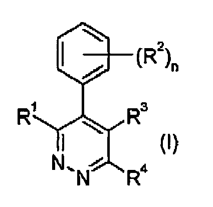 wherein
R¹ is hydrogen, C₁-C₆alkyl, C₁-C₆haloalkyl or C₃-C₆cycloalkyl;
R² is cycloalkyl, cycloalkylalkyl, halocycloalkyl, cycloalkoxy, halocycloalkoxy, cycloalkylalkoxy, halocycloalkylalkoxy, alkoxyalkyl, cycloalkoxyalkoxyalkyl, haloalkoxyalkyl, trialkylsilyl, alkylthioalkyl, haloalkylthioalkyl, cycloalkylthio, halocycloalkylthio, cycloalkylalkylthio, halocycloalkylalkylthio, alkylsulfinylalkyl, alkylsulfonylalkyl, alkylsulfinyl, haloalkylsulfinyl, alkylsulfonyl, haloalkylsulfonyl, optionally substituted arylsulfonyl, optionally substituted aryloxy, optionally substituted heteroaryloxy, optionally substituted arylthio, optionally substituted heteroarylthio; or
R² together with an adjacent carbon atom forms a fused aromatic carbocyclic ring, a fused non aromatic carbocyclic ring, a fused aromatic heterocyclic ring or a fused non aromatic heterocyclic ring;
R³ is an optionally substituted aryl;
R⁴ is hydrogen, halogen, C₁-C₆alkyl, C₁-C₆haloalkyl, C₁-C₆alkoxy, C₁-C₆haloalkoxy, hydroxy or cyano; and
n is a whole number from 1 to 4.

2. The compound according to claim 1 in an agrochemically usable salt form.

3. The compound according to either claims 1 or 2 wherein R¹ is C₁-C₆alkyl or C₁-C₆haloalkyl.

4. The compound according to any one of claims 1 to 3 wherein R² is cycloalkyl, cycloalkylalkyl, halocycloalkyl, cycloalkoxy, cycloalkylalkoxy, alkoxyalkyl, haloalkoxyalkyl, trialkylsilyl, alkylthioalkyl, haloalkylthioalkyl, cycloalkylthio, cycloalkylalkylthio, alkylsulfinyl, haloalkylsuffinyl, alkylsulfonyl, haloalkylsulfonyl, optionally substituted arylsulfonyl, optionally substituted aryloxy, optionally substituted heteroaryloxy, optionally substituted arylthio or optionally substituted heteroarylthio.

5. The compound according to any one of claims 1 to 4 wherein R² together with an adjacent carbon atom forms a fused aromatic carbocyclic ring, a fused non aromatic carbocyclic ring, a fused aromatic heterocyclic ring or a fused non aromatic heterocyclic ring.

6. The compound according to any one of claims 1 to 5 wherein R³ is an optionally substituted aryl.

7. The compound according to any one of claims 1 to 6 wherein R⁴ is halogen, C₁-C₆alkyl, C₁-C₆haloalkyl, C₁-C₆alkoxy, C₁-C₆haloalkoxy or hydroxy.

8. The compound according to any one of claims 1 to 7 wherein
R¹ is C₁-C₆alkyl or C₁-C₆haloalkyl;
R² is cycloalkyl, cycloalkylalkyl, halocycloalkyl, cycloalkoxy, cycloalkylalkoxy, alkoxyalkyl, haloalkoxyalkyl, trialkylsilyl, alkylthioalkyl, haloalkylthioalkyl,cycloalkylthio, cycloalkylalkylthio, alkylsulfinyl, haloalkylsulfinyl, alkylsulfonyl, haloalkylsulfonyl, optionally substituted arylsulfonyl, optionally substituted aryloxy, optionally substituted heteroaryloxy, optionally substituted arylthio or optionally substituted heteroarylthio; or
R² together with an adjacent carbon atom forms a fused aromatic carbocyclic ring, a fused non aromatic carbocyclic ring, a fused aromatic heterocyclic ring or a fused non aromatic heterocyclic ring;
R³ is an optionally substituted aryl;
R⁴ is halogen, C₁-C₆alkyl, C₁-C₆haloalkyl, C₁-C₆alkoxy, C₁-C₆haloalkoxy or hydroxy; and
n is a whole number from 1 to 4.

9. The compound according to any one of claims 1 to 8 wherein
R¹ is C₁-C₃alkyl;
R² is cycloalkyl, halocycloalkyl, cycloalkoxy, alkoxyalkyl, haloalkoxyalkyl, alkylsulfonyl, optionally substituted arylsulfonyl, optionally substituted aryloxy; or
R² together with the adjacent carbon atom forms a fused aromatic carbocyclic ring, a fused non aromatic carbocyclic ring or a fused aromatic heterocyclic ring;
R³ is an optionally substituted aryl;
R⁴ is halogen, C₁-C₃alkyl, C₁-C₃alkoxy or hydroxy; and
n is a whole number from 1 to 3.

10. The compound according to any one of claims 1 to 9 wherein
R¹ is methyl;
R² is cycloalkyl, halocycloalkyl, alkoxyalkyl, haloalkoxyalkyl, alkylsulfonyl, optionally substituted aryloxy; or
R² together with the adjacent carbon atom, forms a fused aromatic carbocyclic ring or a fused non aromatic carbocyclic ring;
R³ is an optionally substituted phenyl;
R⁴ is fluoro, chloro, methyl, methoxy or hydroxy; and
n is a whole number from 1 to 2.

11. A compound selected from
3-chloro-5-(4-cyclopropylphenyl)-6-methyl-4-(2,4,6-trifluorophenyl)-pyridazine,
3-chloro-5-(4-methanesulfonylphenyl)-6-methyl-4-(2,4,6-trifluorophenyl)-pyridazine,
3-chloro-6-methyl-5-naphthalen-2-yl-4-(2,4,6-trifluorophenyl)-pyridazine,
3-chloro-5-[4-(4-chlorophenoxy)-phenyl]-6-methyl-4-(2,4,6-trifluorophenyl)-pyridazine,
3-chloro-6-methyl-5-(4-phenoxyphenyl)-4-(2,4,6-trifluorophenyl)-pyridazine,
3-chloro-6-methyl-5-(4-phenylsulfanylphenyl)-4-(2,4,6-trifluorophenyl)-pyridazine,
3-chloro-5-[4-(4-chlomphenylsulfanyl)-phenyl]-6-methyl-4-(2,4,6-trifluorophenyl)-pyridazine,
3-chloro-6-methyl-5-(4-trifluoromethanesulfonylphenyl)-4-(2,4,6-trifluorophenyl)-pyridazine,
3-chloro-5-(4-cyciopentyloxyphenyi)-6-methyl-4-(2,4,6-trifluorophenyl)-pyridazine,
3-chloro-5-(4-methoxymethylphenyl)-6-methyl-4-(2,4,6-trifluorophenyl)-pyridazine,
3-chloro-6-methyl-5-(4-methylsuffanylmethylphenyl)-4-(2,4,6-trifluorophenyl)-pyridazine, and
3-chloro-5-(4-cyclopropylmethylphenyl)-6-methyl-4-(2,4,6-trifluorophenyl)-pyridazine.

12. A process for the preparation of a compound of formula I.1, 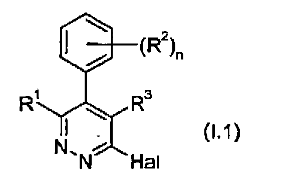 wherein R¹, R², R³ and n are as defined for compound of formula I and Hal is halogen, which comprises reacting a compound of formula I.5, 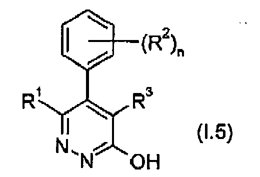 wherein R¹, R², R³ and n are as defined for compound of formula I, with a phosphorus oxyhalide or a thionyl halide.

13. A process for the preparation of a compound of formula I.5, 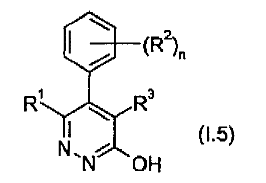 wherein R¹, R², R³ and n are as defined for compound of formula I, which comprises reacting a compound of formula II, 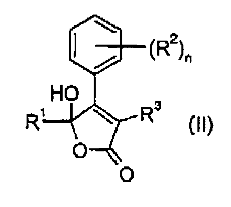 wherein R¹, R², R³ and n are as defined for compound of formula I, with a hydrazine derivative.

14. A process for the preparation of a compound of formula II, 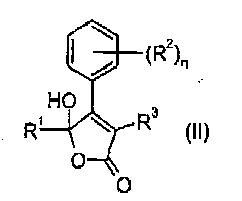 wherein R¹, R², R³ and n are as defined for compound of formula I, which comprises an oxidation with oxygen, air or 3-chloroperbenzoic acid of a compound of formula III, 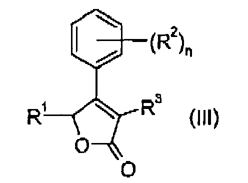 wherein R¹, R², R³ and n are as defined for compound of formula I.

15. A process for the preparation of a compound of formula III, 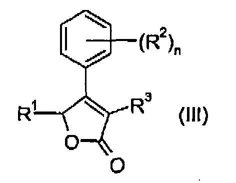 wherein R¹, R², R³ and n are as defined for compound of formula I, which comprises reacting a compound of formule IV, 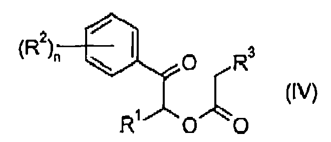 wherein R¹, R², R³ and n are as defined for compound of formula I, with a base.

16. A fungicidal composition for controlling or protecting against phytopathogenic microorganisms, comprising as active ingredient at least one compound as defined in any one of claims 1 to 11, in free form or in agrochemically usable salt form, and at least one adjuvant.

17. The composition according to claim 16, which comprises at least one additional fungicidally active compound, preferably selected from the group consisting of azoles, pyrimidinyl carbinoles, 2-amino-pyrimidines, morpholines, anilinopyrimidines, pyrroles, phenylamides, benzimidazoles, dicarboximides, carboxamides, strobilurines, dithiocarbamates, N-halomethyfthiotatrahydrophthalimides, copper-compounds, nitrophenols, organo-phosphorus-derivatives, pyridazines, triazolopyrimidines or benzamides.

18. The use of a compound as defined in any one of claims 1 to 11 for controlling or preventing infestation of plants, harvested food crops, seeds or non-living materials by phytopathogenic microorganisms.

19. A method of controlling or preventing an infestation of crop plants, harvested food crops or non-living materials by phytopathogenic or spoilage microorganisms or organisms potentially harmful to man, which comprises the application of a compound as defined in any one of claims 1 to 11, as active ingredient to the plant, to parts of the plants or to the locus thereof, to seeds or to any part of the non-living materials.

20. The method according to claim 19, wherein the phytopathogenic microorganisms are fungal organisms.
